# EUROPEAN PATENT APPLICATION

(11) **EP 4 804 199 A1**
(43) Date of publication of application: **09.09.2026**
(21) Application number: 24907896.5
(22) Date of filing: 03.12.2024
(51) Int. Cl.: G16H 20/10, G16H 10/60, A61B 5/00, A61B 5/01, A61B 5/024, A61B 5/145, G06K 19/06, G08B 7/06

(54) **ELECTRONIC DEVICE FOR PROVIDING GUIDELINES RELATED TO MEDICATION AND OPERATION METHOD THEREOF**

(30) Priority: 18.12.2023 KR 20230184501; 02.02.2024 KR 20240016658
(71) Applicant: Samsung Electronics Co., Ltd., Suwon-si, Gyeonggi-do 16677 (KR)
(72) Inventor: KIM, Jieun, Suwon-si Gyeonggi-do 16677 (KR); KIM, Sangtai, Suwon-si Gyeonggi-do 16677 (KR); CHUN, Youngwoon, Suwon-si Gyeonggi-do 16677 (KR); HWANG, Sinyoung, Suwon-si Gyeonggi-do 16677 (KR)
(74) Representative: Gulde & Partner
(86) International application number: PCT/KR2024/019610
(87) International publication number: WO 2025/135612

(57) **Abstract**

Disclosed is an electronic device. The electronic device may comprise: a touch screen; a communication circuit; a first memory for storing instructions; at least one processor; and a second memory. The instructions, when executed by the at least one processor, cause the electronic device to: store, in the second memory as medication information, information about a medicine administered to a ward and information about medication timing acquired via the touch screen; store, in the second memory, first data related to the ward received from an external device via the communication circuit; store, in the second memory, second data related to the ward received from the external device via the communication circuit; identify the amount of change in biometric information about the ward on the basis of the first data and the second data; and provide a notification on the basis of the medication information stored in the second memory and the identified amount of change in biometric information.

## Description

### [Technical Field]

The disclosure relates to an electronic device providing a guide related to medication according to an embodiment and a method of operating the same.

### [Background Art]

With the remarkable advancement of information and communication technology and semiconductor technology, the distribution and use of various electronic devices are rapidly increasing. Electronic devices are being developed so that a user may carry them around and communicate. An electronic device may refer to a device performing a specific function according to a loaded program, such as a mobile communication terminal, a tablet PC, a video/audio device, a desktop/laptop computer, or a vehicle navigation system.

A user may identify information related to a human body, such as a body temperature and a heart rate, using an electronic device. Meanwhile, when a patient (e.g., an infant) is in a state of fever, a parent may continuously monitor the body temperature of the patient to identify the health status of the infant.

The above-described information may be provided as related art for the purpose of helping understanding of the disclosure. No claim or determination is made as to whether any of the foregoing is applicable as background art in relation to the disclosure.

### [Detailed Description of the Invention]

### [Technical Solution]

An electronic device according to an embodiment of the disclosure may include a touchscreen, communication circuitry, first memory storing instructions, at least one processor, and second memory.

According to an embodiment, the instructions may, when executed by the at least one processor, cause the electronic device to store information about a medicine administered to a patient and information of the administration time, obtained through the touchscreen, as medication information in the second memory.

According to an embodiment, the instructions may, when executed by the at least one processor, cause the electronic device to store first data related to the patient, received through the communication circuitry from an external device, in the second memory.

According to an embodiment, the instructions may, when executed by the at least one processor, cause the electronic device to store second data related to the patient, received through the communication circuitry from the external device, in the second memory.

According to an embodiment, the instructions may, when executed by the at least one processor, cause the electronic device to identify an amount of change in biometric information for the patient based on the first data and the second data.

According to an embodiment, the instructions may, when executed by the at least one processor, cause the electronic device to provide a notification based on the medication information stored in the second memory and the identified amount of change in the biometric information.

According to an embodiment of the disclosure, a method of operating an electronic device may include storing information about a medicine administered to a patient and information of the administration time, obtained through a touchscreen, as medication information in second memory.

According to an embodiment, the method may include storing first data related to the patient, received from an external device, in the second memory.

According to an embodiment, the method may include storing second data related to the patient, received from the external device, in the second memory.

According to an embodiment, the method may include identifying an amount of change in biometric information for the patient based on the first data and the second data.

According to an embodiment, the method may include providing a notification based on the medication information stored in the second memory and the identified amount of change in the biometric information.

In a storage medium storing computer-readable instructions according to an embodiment of the disclosure, the instructions may, when executed by at least one processor of an electronic device, cause the electronic device to store information about a medicine administered to a patient and information of the administration time, obtained through the touchscreen, as medication information in the second memory.

According to an embodiment, the instructions may, when executed by the at least one processor, cause the electronic device to store first data related to the patient, received through the communication circuitry from an external device, in the second memory.

According to an embodiment, the instructions may, when executed by the at least one processor, cause the electronic device to store second data related to the patient, received through the communication circuitry from the external device, in the second memory.

According to an embodiment, the instructions may, when executed by the at least one processor, cause the electronic device to identify an amount of change in biometric information for the patient based on the first data and the second data.

According to an embodiment, the instructions may, when executed by the at least one processor, cause the electronic device to provide a notification based on the medication information stored in the second memory and the identified amount of change in the biometric information.

### [Brief Description of Drawings]

FIG. 1 is a block diagram illustrating an electronic device in a network environment according to an embodiment.
FIG. 2 is a block diagram illustrating configurations of an electronic device according to an embodiment.
FIG. 3A is a flowchart illustrating a method of providing a notification related to medication according to an embodiment.
FIG. 3B is a view illustrating a method of providing a notification related to medication according to an embodiment.
FIG. 4 is a flowchart illustrating a method of providing a notification related to medication according to an embodiment.
FIG. 5 is a flowchart illustrating a method of providing a notification related to medication according to an embodiment.
FIG. 6 is a flowchart illustrating a method of providing a notification related to medication according to an embodiment.
FIG. 7 is a flowchart illustrating a method of communicating with an external device according to an embodiment of the disclosure.
FIG. 8 is a view illustrating observation information corresponding to a patient according to an embodiment of the disclosure.
FIG. 9 is a flowchart illustrating a method of displaying guide information based on an estimated body temperature according to an embodiment.
FIG. 10 is a view illustrating a method of obtaining information through a touchscreen according to an embodiment.
FIG. 11 is a view illustrating a method of identifying sensing data based on a period of sensing according to an embodiment.
FIG. 12 is a flowchart illustrating a method of identifying sensing data based on a period of sensing according to an embodiment.
FIG. 13A is a view illustrating a method of identifying identification information of a patient according to an embodiment.
FIG. 13B is a view illustrating a method of identifying identification information of a patient according to an embodiment.
FIG. 13C is a view illustrating a method of providing preset guide information according to an embodiment.
FIG. 14 is a flowchart illustrating a method of providing guide information corresponding to cross-dose according to an embodiment.
FIG. 15 is a view illustrating a method of providing guide information corresponding to cross-dose according to an embodiment.
FIG. 16 is a view illustrating a method of providing history information according to an embodiment.
FIG. 17 is a view illustrating a method of providing preset guide information according to an embodiment.
FIG. 18 is a view illustrating a method of providing preset guide information according to an embodiment.

### [Mode for Carrying out the Invention]

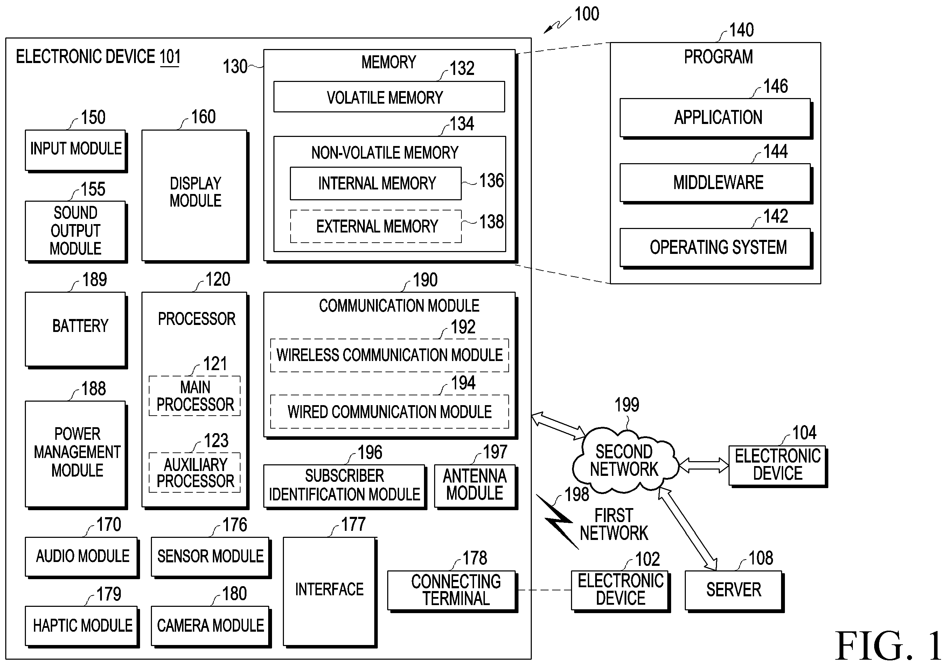

FIG. 1 is a block diagram illustrating an electronic device 101 in a network environment 100 according to various embodiments. Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operation state (e.g., power or temperature) of the electronic device 101 or an external environmental state (e.g., the user's state), and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wiredly) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wiredly) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

In the following description, the components easy to understand from the description of the above embodiments are denoted with or without the same reference numerals and their detailed description may be skipped. According to an embodiment of the disclosure, an electronic device may be implemented by selectively combining configurations of different embodiments, and the configuration of one embodiment may be replaced by the configuration of another embodiment. However, it is noted that the disclosure is not limited to a specific drawing or embodiment.

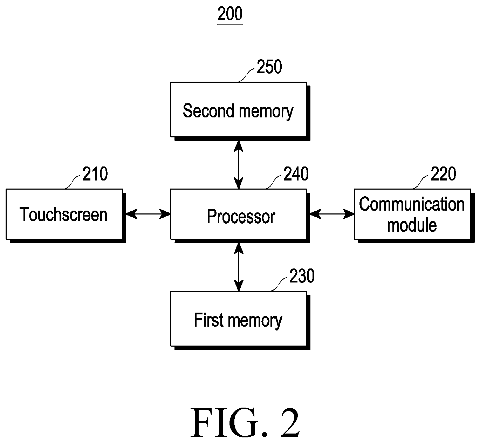

FIG. 2 is a block diagram illustrating configurations of an electronic device according to an embodiment.

Referring to FIG. 2, in an embodiment, an electronic device (e.g., the electronic device 101 of FIG. 1, 200) may include a touchscreen 210, a communication module 220 (e.g., communication circuitry), first memory 230, a processor 240, and/or second memory 250. The electronic device 200 may further include a speaker (e.g., the sound output module 155 of FIG. 1). The electronic device 200 may further include a camera (e.g., the camera module 180 of FIG. 1).

In an embodiment, the touchscreen 210 may be a configuration at least partially identical or similar to the display module 160 of FIG. 1.

In an embodiment, the touchscreen 210 may visually provide information to the outside of the electronic device 101 (e.g., a user). In an embodiment, the touchscreen 210 may visually provide information for receiving a specific input. In an embodiment, the touchscreen 210 may obtain a touch input for obtaining medication information. In an embodiment, the medication information may include information about a medicine taken by a patient (or a medicine administered to a patient) and/or a medicine scheduled to be taken. In an embodiment, the medication information may include at least one of identification information of the patient (e.g., a name of the patient), identification information of the medicine (e.g., a product name of the medicine), a type of the medicine (e.g., acetaminophen or ibuprofen), or information about a time of taking medicine (or the administration time of the medicine). This is described below.

In an embodiment, the communication module 220 may be a configuration at least partially identical or similar to the communication module 190 of FIG. 1.

In an embodiment, the communication module 220 may perform communication with an external device (e.g., the electronic device 102 of FIG. 1, the electronic device 104, or the server 108). In an embodiment, the external device may be implemented as a wearable device. For example, the external device may be implemented as a smart watch or a smart ring, but the disclosure is not limited thereto. This is described below.

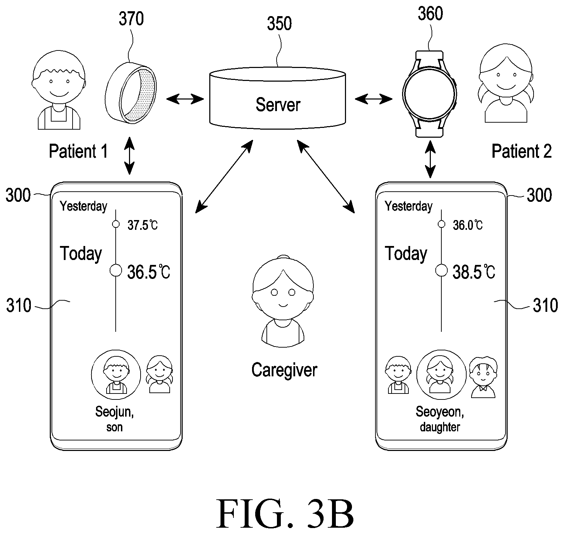

In an embodiment, the communication module 220 may receive sensing data from an external device (e.g., the external device 350, 360, or 370 of FIG. 3B). In an embodiment, the communication module 220 may receive sensing data from a wearable device (e.g., the wearable device 360 or 370 of FIG. 3B) (e.g., a smart watch or a smart ring). In an embodiment, the communication module 220 may also receive sensing data from a server (e.g., the server 350 of FIG. 3B).

In an embodiment, the sensing data may be data related to at least one of a body temperature, a heart rate, or an oxygen saturation. In an embodiment, the sensing data may be bit-type or byte-type data obtained from a sensor module included in a wearable device (e.g., a smart watch or a smart ring), or data in which the obtained data is changed to a specific format. In an embodiment, the communication module 220 may directly receive sensing data from a wearable device. In an embodiment, the communication module 220 may also receive sensing data, transmitted from a wearable device to a server, from the server. The sensing data is described below.

In an embodiment, the sensing data may include first data related to the patient and second data related to the patient. In an embodiment, the communication module 220 may receive the first data related to the patient through an external device (e.g., the external device 350, 360, or 370 of FIG. 3B). In an embodiment, the first data may be sensing data immediately after the patient takes medicine or the first sensing data obtained after taking medicine. However, the disclosure is not limited thereto, and the first data may also be sensing data immediately before the patient takes medicine.

In an embodiment, the communication module 220 may receive the second data related to the patient through an external device (e.g., the external device 350, 360, or 370 of FIG. 3B). In an embodiment, the second data may be sensing data of the patient at a time when a designated time elapses after the patient takes medicine. Alternatively, in an embodiment, the second data may be data related to the patient, received after the first data is received.

In an embodiment, the sensing data may include at least one of data corresponding to each of a body temperature, a heart rate, and an oxygen saturation. In an embodiment, the first data may include at least one of data of the temperature of the patient, data of the heart rate of the patient, or data of the oxygen saturation of the patient. In an embodiment, the second data may include at least one of data of the temperature of the patient, data of the heart rate of the patient, or data of the oxygen saturation of the patient.

In an embodiment, the first memory 230 and/or the second memory 250 may be a configuration at least partially identical or similar to the memory 130 of FIG. 1. For example, the first memory 230 and/or the second memory 250 is for temporarily or permanently storing digital data and may include at least a portion of the configuration and/or function of the memory 130 of FIG. 1.

The first memory 230 and/or the second memory 250 according to an embodiment may store various instructions that may be executed by the processor 240. Further, the first memory 230 and/or the second memory 250 may store at least a portion of the program 140 of FIG. 1. Such instructions may include a control command such as a logical operation and data input/output, which may be recognized and executed by the processor 240. Although there is no limitation on the type and/or amount of data that may be stored in the first memory 230 and/or the second memory 250, in the present document, the configuration and function of the memory related to a method of providing a guide related to medication according to various embodiments and the operation of the processor 240 performing the method is described. The first memory 230 and/or the second memory 250 may store various information, and the various information stored in the first memory 230 and/or the second memory 250 is described below in detail.

In an embodiment, the second memory 250 may store the obtained medication information.

The second memory 250 according to an embodiment may store the first data related to the patient. Alternatively, in an embodiment, the second memory 250 may store the second data related to the patient. In an embodiment, the processor 240 may be a configuration at least partially identical or similar to the processor 120 of FIG. 1. In one embodiment, the processor 240 may include one or more processors.

In an embodiment, the processor 240 may perform various operations by executing the instructions stored in the first memory 230.

In an embodiment, the processor 240 may store the obtained medication information in the first memory 230. In an embodiment, the processor 240 may store the medication information input through the touchscreen 210 in the first memory 230. In an embodiment, the processor 240 may obtain medication information through a quick response (QR) code identified through a camera (e.g., the camera module 180 of FIG. 1), and may store the obtained medication information in the first memory 230. A specific method of obtaining medication information is described below.

In an embodiment, the processor 240 may identify sensing data received from an external device through the communication module 220. For example, the processor 240 may identify sensing data received from a wearable device through the communication module 220. For example, the processor 240 may identify sensing data received from a server through the communication module 220. For example, the processor 240 may identify the first data received from an external device (e.g., a wearable device) through the communication module 220. For example, the processor 240 may identify the second data received from a wearable device through the communication module 220.

In an embodiment, the processor 240 may identify an amount of change in the biometric information based on the received sensing data. In an embodiment, the biometric information may be data obtained by processing the sensing data. In an embodiment, the biometric information may include at least one of a body temperature, a heart rate, and an oxygen saturation. In an embodiment, the amount of change in the biometric information may be an amount of change measured based on the biometric information corresponding to the time of taking medicine. In an embodiment, the amount of change in the biometric information may include increase/decrease information or trend information corresponding to each of the body temperature, the heart rate, and the oxygen saturation. In an embodiment, the processor 240 may identify data corresponding to a body temperature among the received sensing data, and may identify an amount of change in the body temperature based on the identified data, but the disclosure is not limited thereto. The amount of change in the biometric information is described below.

In an embodiment, the processor 240 may identify an amount of change in the biometric information using the first data and the second data. In an embodiment, the amount of change in the biometric information may be an amount of change measured based on the biometric information corresponding to the time of taking medicine. In an embodiment, the biometric information corresponding to the time of taking medicine may be the biometric information corresponding to the first data. In an embodiment, the second data may be data sensed after the first data. In an embodiment, the processor 240 may identify an amount of change in the biometric information corresponding to the patient by comparing the first data and the second data.

In an embodiment, the processor 240 may provide a notification (or a notification related to medication) based on the medication information and the amount of change in the biometric information. In an embodiment, the notification related to medication may include providing (or displaying) a medication guide through the touchscreen 210, providing an audio-type alarm, or providing a vibration-type alarm, but the disclosure is not limited thereto. In an embodiment, the notification related to medication may include information for notifying when the body temperature of the patient goes out of a normal range or is predicted to go out of the normal range. In an embodiment, the notification related to medication may also include information for notifying that a scheduled time for taking medicine has arrived or is about to arrive.

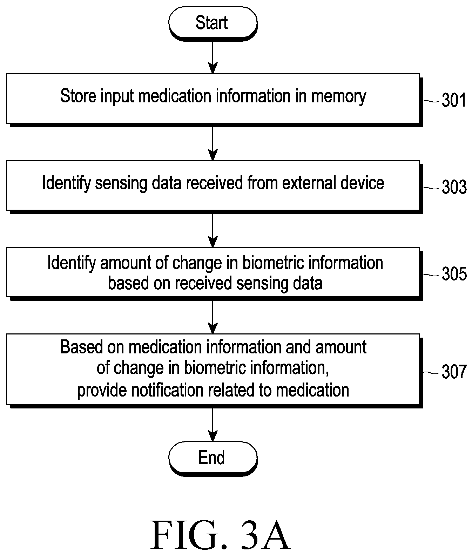

In an embodiment, the processor 240 may display different types of medication guides (or guide information) through the touchscreen 210. For example, when it is identified that an amount of change in the body temperature is greater than or equal to a target value based on the identified amount of change in the biometric information, the processor 240 may display guide information for suggesting taking an antipyretic through the touchscreen 310. Various embodiments of providing a notification related to medication is described below in detail with reference to FIG. 3A.

In an embodiment, the electronic device 200 may further include a speaker (e.g., the sound output module 155 of FIG. 1). In an embodiment, the speaker may output an audio-type alarm corresponding to a first period. In an embodiment, when it is identified that an amount of change in the biometric information satisfies a specific condition, the processor 240 may output a specific type of alarm. This is described below.

In an embodiment, the electronic device 200 may further include a camera (e.g., the camera module 180 of FIG. 1). In an embodiment, the camera may obtain an image including a QR code. In an embodiment, the processor 240 may access an external server (e.g., the server 108 of FIG. 1) storing information about the medicine through the QR code included in the obtained image. In an embodiment, the processor 240 may receive information about the medicine from an external server through the communication module 220. In an embodiment, the information about the medicine may include different types of information including identification information of the medicine (e.g., a product name of the medicine), a type of the medicine (e.g., acetaminophen or ibuprofen), a recommended dosage, and issue information about the medicine, and the information about the medicine is described below.

FIG. 3A is a flowchart illustrating a method of providing a notification related to medication according to an embodiment. FIG. 3B is a view illustrating a method of providing a notification related to medication according to an embodiment.

The method of operating the electronic device 300 according to various embodiments is described in detail. According to various embodiments, the operations performed by the electronic device 300 described below may be executed by a processor (e.g., the processor 240 of FIG. 2) including at least one processing circuitry of the electronic device 300. According to an embodiment, the operations performed by the electronic device 300 may be executed by instructions stored in memory (e.g., the first memory 230 of FIG. 2 and/or the second memory 250) and causing the processor 240 to operate when executed. In the following embodiment, each of the operations may be sequentially performed, but is not necessarily sequentially performed. For example, the order of the operations may be changed, and at least two operations may be performed in parallel. According to the implementation, a specific operation may be omitted.

Referring to FIGS. 3A and 3B, according to an embodiment, in operation 301, the electronic device 300 (e.g., the electronic device 200 of FIG. 2 (e.g., the processor 240)) may store obtained (or received) medication information (e.g., the medication information of FIG. 2) in memory (e.g., the second memory 250 of FIG. 2). The medication information may include information about a medicine taken by the patient or a medicine scheduled to be taken. In an embodiment, the medication information may include at least one of identification information of the patient (e.g., a name of the patient), identification information of the medicine (e.g., a product name of the medicine), a type of the medicine (e.g., acetaminophen or ibuprofen), or information about the time of taking medicine.

In an embodiment, the medication information may be input through the touchscreen 310 (e.g., the touchscreen 210 of FIG. 2). In an embodiment, the electronic device 300 may obtain the medication information based on an input (e.g., a touch input) obtained through the touchscreen 310. In an embodiment, the electronic device 300 may store the obtained medication information in the memory.

In an embodiment, the medication information may also be obtained based on a QR code. In an embodiment, the electronic device 300 may include a camera (e.g., the camera module 180 of FIG. 1), and the electronic device 300 may identify the QR code through the camera. In an embodiment, the electronic device 300 may access an external server (e.g., the server 108 of FIG. 1) storing information about the medicine through the QR code. In an embodiment, the electronic device may receive information about the medicine from an external server through a communication module. In an embodiment, the information about the medicine may include different types of information including identification information of the medicine (e.g., a product name of the medicine), a type of the medicine (e.g., acetaminophen or ibuprofen), a recommended dosage, and issue information about the medicine, and the information about the medicine is described below.

In an embodiment, when an input (e.g., a user input for the time of taking medicine) through the touchscreen is received, the electronic device 300 may also identify the medication information based on the information about the medicine obtained through the QR code and/or the input through the touchscreen. For example, the electronic device 300 may identify that the medicine (e.g., Brufen syrup) obtained through the QR code was taken at the time of taking medicine input by the user. The electronic device 300 may identify this as the medication information. In an embodiment, the information about the time of taking medicine may be automatically set to the time when the user completed the input of information related to medication through the execution screen as described above, or the time of taking medicine may be set by the user directly inputting the time of taking medicine.

According to an embodiment, in operation 303, the electronic device 300 may identify sensing data received from an external device 350, 360, or 370 (e.g., the electronic device 102 of FIG. 1, the electronic device 104, or the server 108). In an embodiment, the electronic device 300 may receive sensing data from the external device 350, 360, or 370 through a communication module (e.g., the communication module 220 of FIG. 2).

In an embodiment, the sensing data may include at least one of data corresponding to each of a body temperature, a heart rate, and an oxygen saturation. In an embodiment, the sensing data may be bit-type or byte-type data obtained from a sensor module included in a wearable device 360 or 370 (e.g., a smart watch or a smart ring), or data in which the obtained data is changed to a specific format.

In an embodiment, the sensing data may include first data related to the patient and second data related to the patient. In an embodiment, the electronic device 300 may receive the first data related to the patient through the communication module 220. In an embodiment, the first data may be sensing data immediately after the patient takes medicine or the first sensing data obtained after taking medicine. However, the disclosure is not limited thereto, and the first data may also be sensing data immediately before the patient takes medicine.

In an embodiment, the electronic device 300 may receive the second data related to the patient through the communication module 220 after the first data is received. In an embodiment, the second data may be sensing data of the patient at a time when a designated time elapses after the patient takes medicine. Alternatively, in an embodiment, the second data may be data related to the patient, received after the first data is received. In an embodiment, the wearable device 360 or 370 may obtain sensing data related to at least one of a body temperature, a heart rate, or an oxygen saturation through a sensor module. In an embodiment, the wearable device 360 or 370 may include at least one type of sensor module. In an embodiment, the sensor module included in the wearable device 360 or 370 may be a configuration at least partially identical or similar to the sensor module 176 of FIG. 1. For example, the sensor module included in the wearable device 360 or 370 may include at least one of a temperature sensor (e.g., a contact-type temperature sensor or a non-contact-type temperature sensor), a heart rate measurement sensor (e.g., a photoplethysmogram (PPG) sensor), or an oxygen saturation measurement sensor, but the disclosure is not limited thereto.

In an embodiment, the electronic device 300 may identify sensing data received from the wearable device 360 or 370 corresponding to a patient (e.g., patient 1 or patient 2). In an embodiment, the electronic device 300 may communicate with the wearable device 360 or 370 through the first network 198 of FIG. 1 (e.g., a short-range wireless communication network), or communicate with the wearable device 360 or 370 through the second network 199 of FIG. 1 (e.g., a long-range wireless communication network).

In an embodiment, the electronic device 300 may also identify sensing data received from the server 350. In an embodiment, the electronic device 300 may receive sensing data, transmitted from the wearable device 360 or 370 corresponding to a patient (e.g., patient 1 or patient 2) to the server 350, from the server 350. In an embodiment, the electronic device 300 may communicate with the server 350 through the second network 199 of FIG. 1 (e.g., a long-range wireless communication network).

In an embodiment, the electronic device 300 may store the received sensing data in the memory (e.g., the second memory 250 of FIG. 2). In an embodiment, the electronic device 300 may store the identified first data in the second memory. In an embodiment, the electronic device 300 may store the identified second data in the second memory.

According to an embodiment, in operation 305, the electronic device 300 may identify an amount of change in the biometric information based on the received sensing data. In an embodiment, the biometric information may be data obtained by processing the sensing data. In an embodiment, the biometric information may include at least one of a body temperature, a heart rate, and an oxygen saturation. In an embodiment, the amount of change in the biometric information may be an amount of change measured based on the biometric information corresponding to the time of taking medicine. In an embodiment, the amount of change in the biometric information may include increase/decrease information or trend information corresponding to each of the body temperature, the heart rate, and the oxygen saturation.

However, the disclosure is not limited thereto, and in an embodiment, the amount of change in the biometric information may also be an amount of change measured based on a basal temperature corresponding to a user wearing a wearable device. In an embodiment, the basal temperature may be a personalized body temperature of the user wearing the wearable device. In an embodiment, the basal temperature may be a value calculated based on sensing data corresponding to the user wearing the wearable device. For example, the basal temperature may be an average value of the sensing data corresponding to the user during a specific period (e.g., one week), but the disclosure is not limited thereto.

In an embodiment, the electronic device 300 may identify data corresponding to a body temperature among the received sensing data, and may identify an amount of change in the body temperature based on the identified data. In an embodiment, the electronic device 300 may also identify data corresponding to each of a heart rate or an oxygen saturation among the received sensing data, and may identify each of an amount of change in the heart rate or an amount of change in the oxygen saturation based on the identified data.

In an embodiment, the electronic device 300 may identify an amount of change in the biometric information based on the identified first data and the identified second data. In an embodiment, the biometric information corresponding to the time of taking medicine may be the biometric information corresponding to the first data. In an embodiment, the second data may be data sensed after the first data. In an embodiment, the processor 240 may identify an amount of change in the biometric information corresponding to the patient by comparing the first data and the second data. In an embodiment, the amount of change in the biometric information may be a value calculated based on the biometric information corresponding to the first data. However, the disclosure is not limited thereto.

For example, the electronic device 300 may identify an amount of change in the body temperature corresponding to the patient by comparing data corresponding to the body temperature of the patient among the identified first data with data corresponding to the body temperature of the patient among the identified second data.

According to an embodiment, in operation 307, the electronic device 300 may provide a notification (or a notification related to medication) based on the medication information and the amount of change in the biometric information. In an embodiment, the notification related to medication may include information for notifying when the body temperature of the patient goes out of a normal range or is predicted to go out of the normal range. In an embodiment, the notification related to medication may also include information for notifying that a scheduled time for taking medicine has arrived or is about to arrive. In an embodiment, the notification related to medication may include providing (or displaying) a medication guide through the touchscreen 310, providing an audio-type alarm, or providing a vibration-type alarm, but the disclosure is not limited thereto.

In an embodiment, different types of medication guides (or guide information) may be provided through the touchscreen 310 based on whether a specific condition is satisfied. For example, when it is identified that an amount of change in the body temperature is greater than or equal to a target value based on the identified amount of change in the biometric information, guide information for suggesting taking an antipyretic may be provided through the touchscreen 310. Various embodiments of providing a notification related to medication is described below.

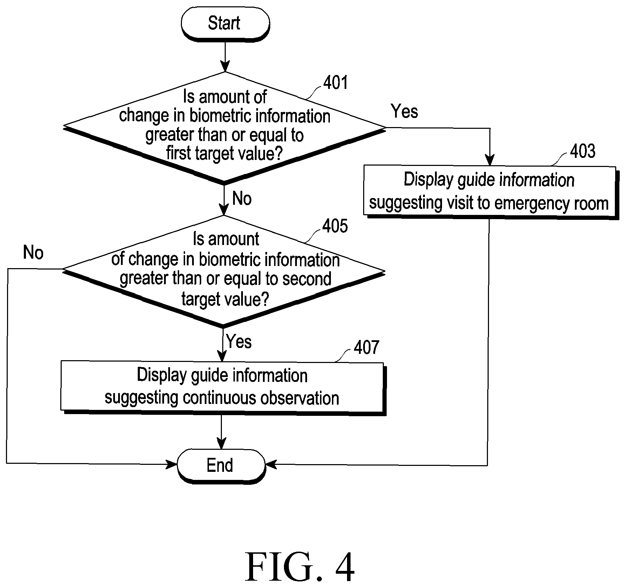

FIG. 4 is a flowchart illustrating a method of providing a notification related to medication according to an embodiment.

Referring to FIG. 4, according to an embodiment, in operation 401, an electronic device (e.g., the electronic device 200 of FIG. 2 (e.g., the processor 240)) may identify whether an amount of change in the biometric information (e.g., the biometric information of FIG. 3A) is greater than or equal to a first target value.

In an embodiment, the electronic device may identify an amount of change in the biometric information (e.g., an amount of change in the body temperature) based on the biometric information corresponding to a reference time. In an embodiment, the reference time may be a time corresponding to comparison data (or reference data) for measuring the amount of change. For example, the electronic device may identify biometric information corresponding to the time of taking medicine based on information about the time of taking medicine input through the touchscreen (e.g., the touchscreen 210 of FIG. 2). The electronic device may identify biometric information corresponding to a reference time based on the identified biometric information corresponding to the time of taking medicine. The electronic device may identify an amount of change in the biometric information by comparing biometric information identified after the time of taking medicine with the biometric information corresponding to the reference time. However, the disclosure is not limited thereto, and for example, a reference value for identifying an amount of change in the biometric information may be stored in the memory (e.g., the first memory 230 of FIG. 2 or the second memory 250 of FIG. 2). The electronic device may also identify an amount of change in the biometric information by comparing the reference value stored in the memory with the identified biometric information. For example, the reference value for identifying an amount of change in the biometric information may also be obtained through an input (e.g., a user input).

In an embodiment, when an amount of change in the biometric information is identified, the electronic device may identify whether the amount of change in the biometric information is greater than or equal to the first target value by comparing the identified amount of change with the first target value.

According to an embodiment, in operation 403, when the amount of change in the biometric information is identified as greater than or equal to the first target value (operation 401 - YES), the electronic device may display guide information for suggesting a visit to an emergency room through the touchscreen.

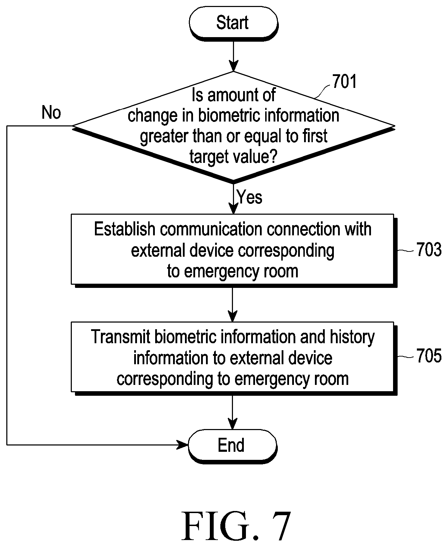

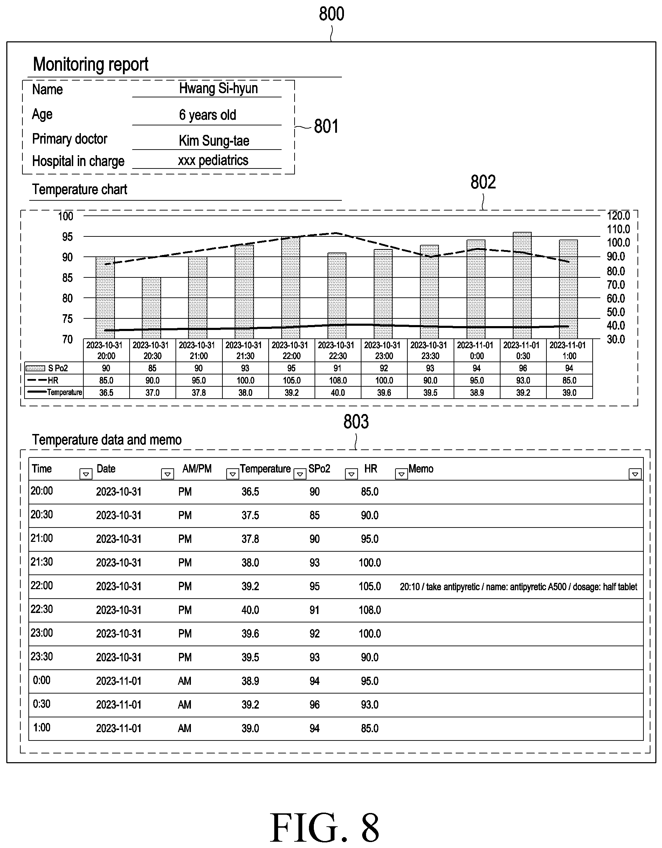

In an embodiment, the electronic device may display guide information for suggesting a visit to an emergency room based on a time during which the amount of change in the biometric information is maintained greater than or equal to the first target value. For example, when it is identified that the amount of change in the biometric information is maintained greater than or equal to the first target value for 5 minutes or more, the electronic device may display guide information for suggesting a visit to an emergency room through the touchscreen. In an embodiment, the electronic device may display the guide information and communicate with an external device (e.g., the electronic device 102 of FIG. 1, the electronic device 104, or the server 108) corresponding to an emergency room. This is described in detail with reference to FIGS. 7 and 8.

According to an embodiment, in operation 405, when the amount of change in the biometric information is identified as less than the first target value (operation 401 - NO), the electronic device may identify whether the amount of change in the biometric information is greater than or equal to a second target value. In an embodiment, the size of the first target value may be set to a value greater than the second target value. In an embodiment, the electronic device may identify an amount of change in the biometric information based on the biometric information corresponding to the time of taking medicine, and may identify whether the amount of change in the biometric information is greater than or equal to the second target value based thereon.

According to an embodiment, in operation 407, when the amount of change in the biometric information is identified as less than the first target value and greater than or equal to the second target value (operation 405 - YES), the electronic device may display guide information for suggesting continuous observation through the touchscreen. In an embodiment, the electronic device may display guide information for suggesting continuous observation of the patient based on a time during which the state in which the amount of change in the biometric information is less than the first target value and greater than or equal to the second target value is maintained.

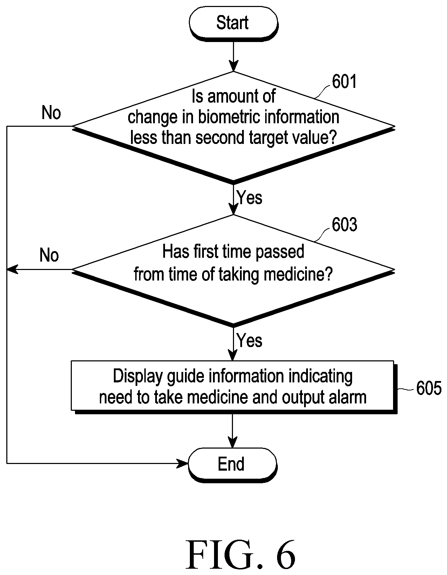

According to an embodiment, when the amount of change in the biometric information is identified as less than the second target value (operation 405 - NO), the electronic device may identify whether a first time has passed from the time of taking medicine. This is described in detail with reference to FIG. 6.

According to the above-described example, the electronic device may provide different types of guide information according to the size of the amount of change in the biometric information. For example, when the amount of change in the body temperature is greater than or equal to the first target value (e.g., a body temperature of 3°C), it may be determined that the state of the patient is urgent, and a guide may be provided to promptly visit an emergency room. Alternatively, when the amount of change in the body temperature is less than the first target value, but greater than or equal to the second target value (e.g., a body temperature of 2.5°C), a guide may be provided to carefully identify the state of the patient.

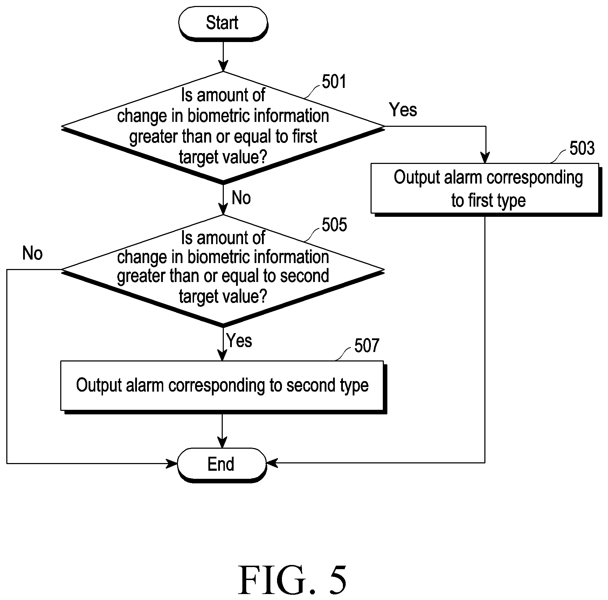

FIG. 5 is a flowchart illustrating a method of providing a notification related to medication according to an embodiment.

Referring to FIG. 5, according to an embodiment, in operation 501, an electronic device (e.g., the electronic device 200 of FIG. 2 (e.g., the processor 240)) may identify whether an amount of change in the biometric information (e.g., the biometric information of FIG. 3A) is greater than or equal to a first target value (e.g., the first target value of FIG. 4). In an embodiment, as described with reference to FIG. 4, when an amount of change in the biometric information (e.g., a body temperature) is identified, the electronic device may identify whether the amount of change in the biometric information is greater than or equal to the first target value by comparing the identified amount of change with the first target value.

According to an embodiment, in operation 503, when the amount of change in the biometric information is identified as greater than or equal to the first target value (operation 501 - YES), the electronic device may output a first alarm corresponding to a first type (e.g., a first size, a first pattern, a first sound, or a first period). In an embodiment, the electronic device may include a speaker (e.g., the sound output module 155 of FIG. 1), and the electronic device may output an audio-type alarm corresponding to the first period through the speaker. For example, the electronic device may output the first alarm corresponding to the first period every set first period. In an embodiment, the electronic device may also output a vibration-type first alarm corresponding to the first period through a haptic module (e.g., the haptic module 179 of FIG. 1).

In an embodiment, the electronic device may output an alarm corresponding to the first period based on a time during which the amount of change in the biometric information is maintained greater than or equal to the first target value. For example, when it is identified that the amount of change in the biometric information is maintained greater than or equal to the first target value for 5 minutes or more, the electronic device may output an alarm corresponding to the first period. However, the disclosure is not limited thereto, and the time during which the amount of change is maintained greater than or equal to the first target value may be different.

According to an embodiment, in operation 505, as illustrated in FIG. 4 (e.g., operation 405), when the amount of change in the biometric information is identified as less than the first target value (operation 501 - NO), the electronic device may identify whether the amount of change in the biometric information is greater than or equal to a second target value (e.g., the second target value of FIG. 4). In an embodiment, the size of the first target value may be set to a value greater than the second target value.

According to an embodiment, in operation 507, when the amount of change in the biometric information is identified as less than the first target value and greater than or equal to the second target value (operation 505 - YES), the electronic device may output a second alarm corresponding to a second type (e.g., a second size, a second pattern, a second sound, or a second period). In an embodiment, the interval of the first period may be set to be smaller than the interval of the second period. In an embodiment, the electronic device may output an audio-type alarm corresponding to the second period through the speaker. For example, the electronic device may output the second alarm corresponding to the second period every set second period. In an embodiment, the electronic device may also output a vibration-type alarm corresponding to the second period through a haptic module.

According to an embodiment, when the amount of change in the biometric information is identified as less than the second target value (operation 505 - NO), the electronic device may identify whether a first time has passed from the time of taking medicine. This is described in detail with reference to FIG. 6.

According to an embodiment, the electronic device may provide the guide information illustrated in FIG. 4 along with an alarm. In an embodiment, when the amount of change in the biometric information is identified as greater than or equal to the first target value, the electronic device may display guide information for suggesting a visit to an emergency room through the touchscreen, and may output an alarm corresponding to the first period through the speaker. In an embodiment, when the amount of change in the biometric information is identified as less than the first target value and greater than or equal to the second target value, the electronic device may also display guide information for suggesting continuous observation of the patient through the touchscreen, and output an alarm corresponding to the second period through the speaker.

According to the above-described example, the electronic device may provide alarms of different periods according to the size of the amount of change in the biometric information. For example, when the amount of change in the body temperature is greater than or equal to the first target value (e.g., a body temperature of 3°C), it may be determined that the state of the patient is urgent, and an alarm of the first period may be output to notify that the circumstance is urgent. Alternatively, when the amount of change in the body temperature is less than the first target value, but greater than or equal to the second target value (e.g., a body temperature of 2.5°C), an alarm of the second period greater than the first period may be output.

FIG. 6 is a flowchart illustrating a method of providing a notification related to medication according to an embodiment.

Referring to FIG. 6, in operation 601, as illustrated in FIG. 4 (e.g., operation 405), an electronic device (e.g., the electronic device 200 of FIG. 2 (e.g., the processor 240)) according to an embodiment may identify whether an amount of change in the biometric information (e.g., the biometric information of FIG. 3A) is less than a second target value (e.g., the second target value of FIG. 4).

According to an embodiment, when the amount of change in the biometric information is identified as less than the second target value (operation 601 - YES), the electronic device may identify, in operation 603, whether a first time has passed from the time of taking medicine. In an embodiment, the electronic device may identify whether the first time (e.g., 2 hours) has passed from the time of taking medicine based on information about the time of taking medicine included in the input medication information (e.g., the medication information of FIG. 2).

According to an embodiment, when the amount of change in the biometric information is identified as greater than or equal to the second target value (operation 601 - NO), the electronic device may identify whether an amount of change in the biometric information (e.g., the biometric information of FIG. 3A) is greater than or equal to a first target value (e.g., the first target value of FIG. 4). In an embodiment, when the amount of change in the biometric information is identified as greater than or equal to the second target value (operation 601 - NO), the electronic device may also display guide information for suggesting continuous observation as illustrated in FIG. 4 (e.g., operation 407).

According to an embodiment, when it is identified that the first time has passed from the time of taking medicine (operation 603 - YES), the electronic device may display, in operation 605, guide information for indicating a need to take medicine through the touchscreen (e.g., the touchscreen 220 of FIG. 2), and may output an audio-type alarm through a speaker (e.g., the sound output module 155 of FIG. 1). In an embodiment, the electronic device may also output a vibration-type alarm through a haptic module (e.g., the haptic module 179 of FIG. 1).

According to an embodiment, when it is identified that the first time has not passed from the time of taking medicine (operation 603 - NO), the electronic device may identify whether the amount of change in the biometric information is greater than or equal to a target value (e.g., the first target value or the second target value).

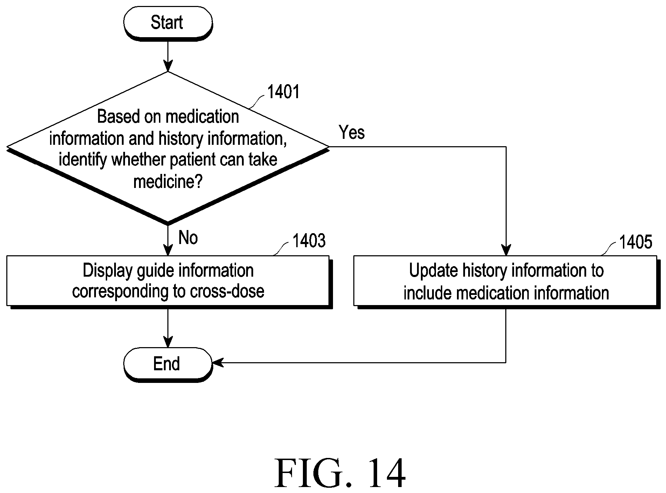

In an embodiment, the electronic device may display at least one of the biometric information or guide information corresponding to cross-dose through the touchscreen along with the guide information for indicating a need to take medicine. For example, the electronic device may display an amount of change in the body temperature corresponding to the patient over time along with the guide information for indicating a need to take medicine. For example, the electronic device may display guide information corresponding to cross-dose through the touchscreen along with the guide information for indicating a need to take medicine, and the guide information corresponding to cross-dose is described in detail with reference to FIG. 14.

Although not illustrated in FIG. 6, the electronic device according to an embodiment may also provide a notification related to medication when the amount of change in the biometric information is identified as less than the second target value, but the first time has not passed from the time of taking medicine.

In an embodiment, when the amount of change in the biometric information is identified as less than the second target value, but the first time has not passed from the time of taking medicine, the electronic device may identify whether a second time has passed from the time of taking medicine. In an embodiment, the second time (e.g., 1 hour) may be less than the first time (e.g., 2 hours). For example, when the amount of change in the body temperature is less than 2.5°C and 2 hours have not passed from the time of taking medicine, the electronic device may identify whether 1 hour has passed from the time of taking medicine.

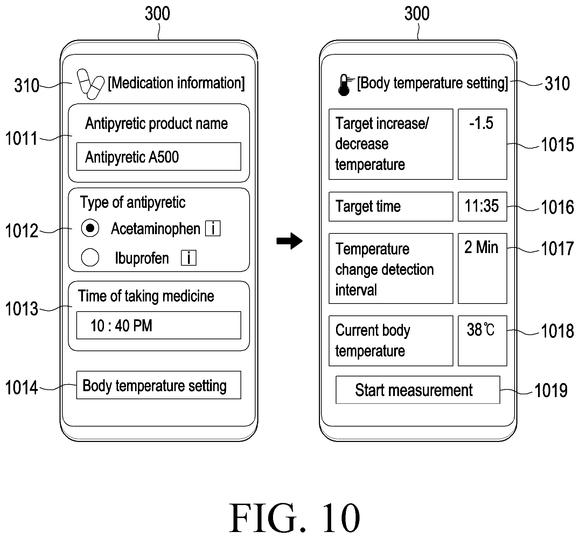

In an embodiment, when it is identified that the second time has passed, the electronic device may identify whether the amount of change in the biometric information is greater than or equal to a designated first value. In an embodiment, the designated first value may be a value obtained by multiplying a third target value by a designated ratio (e.g., 50%). The third target value is a value set by the user as a target amount of change (e.g., a target increase/decrease temperature). For example, a case where the third target value is -1.5°C is assumed. The electronic device may identify whether the amount of change in the body temperature is greater than or equal to the designated first value, which is -0.75°C. The third target value is described in detail with reference to FIG. 10.

In an embodiment, when it is identified that the amount of change in the biometric information is greater than or equal to the designated first value, the electronic device may display guide information for suggesting continuous observation of the patient through the touchscreen as in operation 407 of FIG. 4.

According to the above-described example, the electronic device may notify the user that medicine needs to be taken when a specific time has passed from the time when the patient took medicine, even when the body temperature of the patient is not at a dangerous level. Further, even when a specific time has not passed from the time when the patient previously took medicine, the electronic device may suggest continuous observation of the patient when determining that the body temperature of the patient has not fallen below an appropriate level.

FIG. 7 is a flowchart illustrating a method of communicating with an external device according to an embodiment of the disclosure. FIG. 8 is a view illustrating observation information corresponding to a patient according to an embodiment of the disclosure.

Referring to FIGS. 7 and 8, an electronic device (e.g., the electronic device 200 of FIG. 2 (e.g., the processor 240)) according to an embodiment may identify, in operation 701, as in operation 401 of FIG. 4, whether an amount of change in the biometric information (e.g., the biometric information of FIG. 3A) is greater than or equal to a first target value (e.g., the first target value of FIG. 4).

When the amount of change in the biometric information is identified as greater than or equal to the first target value (operation 701 - YES), the electronic device according to an embodiment may establish, in operation 703, a communication connection to an external device (e.g., the server 108 of FIG. 1) corresponding to an emergency room through a communication module (e.g., the communication module 220 of FIG. 2).

When the amount of change in the biometric information is identified as less than the first target value (operation 701 - NO), the electronic device according to an embodiment may identify whether the amount of change in the biometric information is greater than or equal to the second target value as illustrated in FIG. 4.

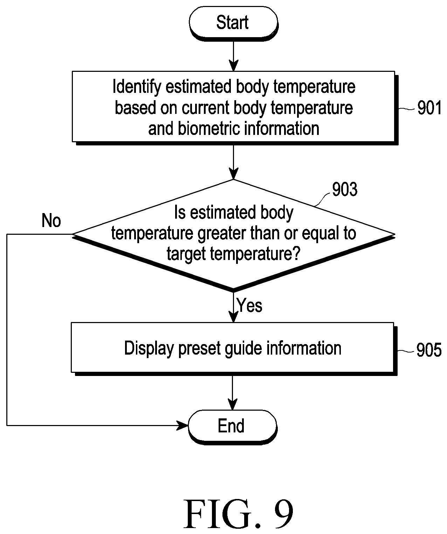

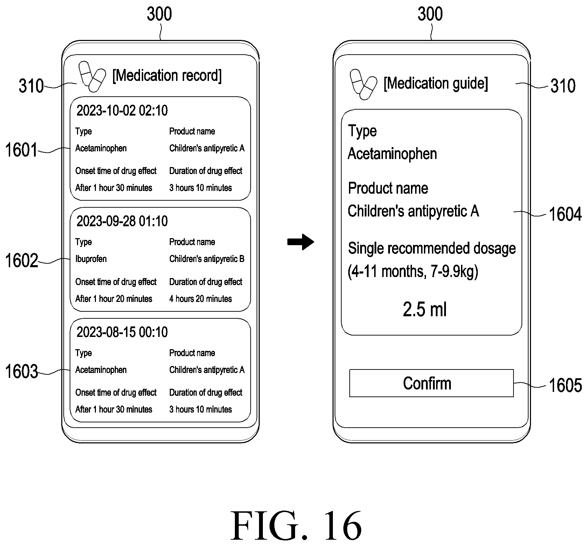

The electronic device according to an embodiment may transmit, in operation 705, the biometric information and history information related to medication (hereinafter, history information) to an external device (e.g., a server) corresponding to an emergency room through the communication module. In an embodiment, the biometric information transmitted to the server corresponding to an emergency room may be information corrected based on the current body temperature of the patient. In an embodiment, the corrected information may be estimated biometric information corrected based on the sensing data and the current biometric information of the patient, and this is described in detail with reference to FIG. 9. In an embodiment, the history information may include information about at least one medication event corresponding to the patient. In an embodiment, the information about the medication event may include at least one of information about a type of medication, a type of the medicine, a time of taking medicine, a time when the medicine takes effect, and a duration of effect of the medicine, but the disclosure is not limited thereto. The history information is described in detail with reference to FIG. 16.

Referring to FIG. 8, the electronic device according to an embodiment may identify observation information 800 corresponding to the patient based on the biometric information and history information (e.g., the history information of FIG. 7), and may transmit the identified observation information 800 to an external device through the communication module.

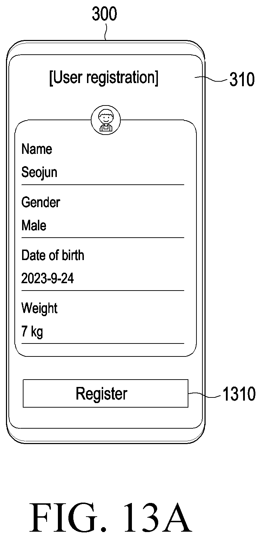

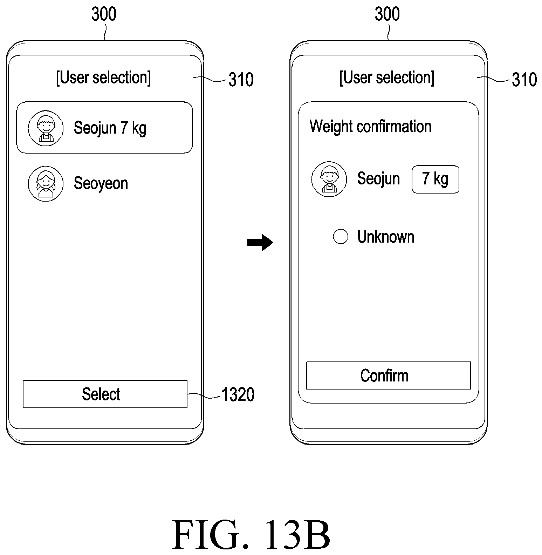

The observation information 800 according to an embodiment may include at least one of identification information 801 of the patient, first information 802, and second information 803. In an embodiment, the identification information 801 of the patient may include information about a name, an age, a primary doctor, and a hospital in charge of the patient. In an embodiment, the first information 802 may include the biometric information (e.g., a body temperature, a heart rate (HR), and an oxygen saturation (SPo2)) corresponding to the patient. In an embodiment, the first information 802 may include graph-type biometric information. In an embodiment, the second information 803 may include a specific type of biometric information (e.g., a body temperature, a heart rate (HR), and an oxygen saturation (Spo2)). In an embodiment, the second information 803 may include biometric information measured every specific time period (e.g., 30 minutes). In an embodiment, the second information 803 may also include history information corresponding to the patient. For example, the second information 803 may include information (e.g., a time of taking medicine, a type of the medicine, identification information of the medicine, and a dosage) about a medication event (e.g., a memo) corresponding to the patient. For example, the medication event corresponding to the patient may be provided to the user based on a specific time period. Meanwhile, a method of obtaining the identification information 801 of the patient is described in detail with reference to FIGS. 13A and 13B.

In an embodiment, the electronic device may also attempt a call connection to a server corresponding to an emergency room through the communication module.

FIG. 9 is a flowchart illustrating a method of displaying guide information based on an estimated body temperature according to an embodiment.

Referring to FIG. 9, an electronic device (e.g., the electronic device 200 of FIG. 2 (e.g., the processor 240)) according to an embodiment may identify, in operation 901, an estimated body temperature based on a body temperature (or a current body temperature) input through a touchscreen (e.g., the touchscreen 210 of FIG. 2) and the biometric information (e.g., the biometric information of FIG. 3A). The current body temperature is a body temperature of the patient measured by a caregiver using a thermometer at the time of taking medicine, and in an embodiment, the electronic device may obtain the current body temperature based on a user input. A specific method of obtaining the current body temperature is described in detail with reference to FIG. 10.

In an embodiment, the electronic device may obtain a correction value by comparing the current body temperature measured using a thermometer at the time of taking medicine and the body temperature corresponding to sensing data received from a wearable device (e.g., the wearable device 360 or 370 of FIG. 3B) at the time of taking medicine. For example, the electronic device may identify a body temperature (e.g., 37.5°C) corresponding to the time of taking medicine among the biometric information identified based on the sensing data received from a wearable device, and may obtain a correction value (e.g., 0.5°C) by comparing the body temperature corresponding to the time of taking medicine with the current body temperature (38°C) input by the user.

In an embodiment, the electronic device may identify the estimated body temperature based on the obtained correction value and the biometric information. For example, when the correction value is obtained, the electronic device may identify the estimated body temperature by adding the obtained correction value to the biometric information. For example, when the correction value is 0.5°C, the estimated body temperature may be information in which 0.5°C is added to the existing biometric information.

However, the disclosure is not limited thereto, and the electronic device according to an embodiment may also identify an estimated heart rate based on a current heart rate and the biometric information (e.g., a heart rate). The electronic device according to an embodiment may also identify an estimated oxygen saturation based on a current oxygen saturation and the biometric information (e.g., an oxygen saturation). The current heart rate is a heart rate of the patient measured by a caregiver, and the current oxygen saturation is also an oxygen saturation of the patient measured by a caregiver.

The electronic device according to an embodiment may identify, in operation 903, whether the estimated body temperature is greater than or equal to a target temperature. In an embodiment, the electronic device may identify whether the estimated body temperature is greater than or equal to a target temperature (e.g., 39.5°C or 39°C) by comparing the size of the identified estimated body temperature with the target temperature. However, the disclosure is not limited thereto, and in an embodiment, the electronic device may also identify whether an amount of change in the estimated body temperature is greater than or equal to a designated target value (e.g., a first target value (e.g., the first target value of FIG. 4) or a second target value (e.g., the second target value of FIG. 4)).

When it is identified that the estimated body temperature is greater than or equal to the target temperature (operation 903 - YES), the electronic device according to an embodiment may display, in operation 905, preset guide information through a touchscreen (e.g., the touchscreen 210 of FIG. 2).

In an embodiment, when it is identified that the estimated body temperature (e.g., 39.6°C) is greater than or equal to a first target temperature (e.g., 39.5°C), the electronic device may display guide information for suggesting a visit to an emergency room through the touchscreen. In an embodiment, the electronic device may also display guide information for suggesting a visit to an emergency room when the time during which the current temperature is maintained greater than or equal to the first target temperature is greater than or equal to a designated time (e.g., 5 minutes).

In an embodiment, when it is identified that the estimated body temperature (e.g., 39.2°C) is less than the first target temperature (e.g., 39.5°C) and greater than or equal to a second target temperature (e.g., 39°C), the electronic device may display guide information for suggesting continuous observation of the patient through the touchscreen. In an embodiment, the electronic device may also display guide information for suggesting continuous observation of the patient when the time during which the current temperature is maintained greater than or equal to the second target temperature is greater than or equal to a designated time (e.g., 5 minutes).

When it is identified that the estimated body temperature is less than the target temperature (operation 903 - NO), the electronic device according to an embodiment may identify whether the first time has passed from the time of taking medicine as illustrated in FIG. 6.

FIG. 10 is a view illustrating a method of obtaining information through a touchscreen according to an embodiment.

Referring to FIG. 10, an electronic device 300 (e.g., the electronic device 200 of FIG. 2 (e.g., the processor 240)) according to an embodiment may display an execution screen including at least one of objects 1011, 1012, 1013, 1014, 1015, 1016, 1017, 1018, and 1019 through a touchscreen 310 (e.g., the touchscreen 210 of FIG. 2).

In an embodiment, the execution screen may display an execution screen (e.g., the left screen of FIG. 10) for obtaining medication information (e.g., the medication information of FIG. 2) corresponding to the patient. In an embodiment, the medication information may include information about a medicine taken by the patient or a medicine scheduled to be taken. In an embodiment, the execution screen may include an object 1011 corresponding to identification information of the medicine. In an embodiment, the electronic device may obtain identification information of the medicine based on an input (e.g., a touch input or a voice input) corresponding to the object 1011. In an embodiment, the execution screen may include an object 1012 corresponding to a type of the medicine (e.g., acetaminophen or ibuprofen). In an embodiment, the electronic device may obtain information about a type of the medicine based on an input (e.g., a touch input) corresponding to the object 1012. In an embodiment, the execution screen may include an object 1013 corresponding to a time of taking medicine, and the electronic device may obtain information about a time of taking medicine based on an input (e.g., a touch input or a voice input) corresponding to the object 1013. In an embodiment, the electronic device may automatically display information of the current time measured by the electronic device in the object 1013 corresponding to the time of taking medicine. When the user completes the input of medication information through the execution screen for obtaining medication information (e.g., the medication information of FIG. 2), the time of taking medicine may be set to the displayed time. In an embodiment, the electronic device may obtain medication information including at least one of identification information of the patient (e.g., a name of the patient), identification information of the medicine (e.g., a product name of the medicine), and information about a time of taking medicine based on an input corresponding to the execution screen.

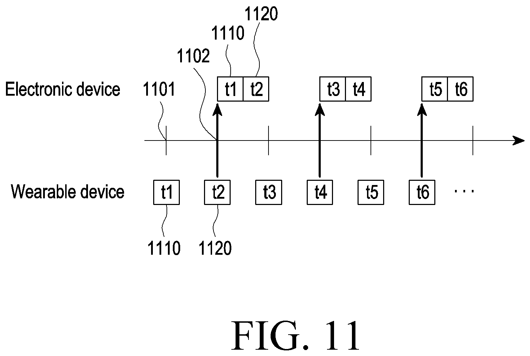

The electronic device according to an embodiment may display an execution screen (the right screen of FIG. 10) for obtaining body temperature setting information corresponding to the patient. In an embodiment, when a touch input for the object 1014 for displaying an execution screen corresponding to body temperature setting information is identified in the left screen of FIG. 10, the electronic device 300 may display an execution screen for obtaining body temperature setting information as in the right screen of FIG. 10. In an embodiment, the body temperature setting information may include at least one of information about a third target value (e.g., the third target value of FIG. 6), a target time, a period of sensing, and a current body temperature. The period of sensing is described in detail with reference to FIG. 11.

In an embodiment, the execution screen may include at least one of an object 1015 corresponding to the third target value (e.g., a target increase/decrease temperature), an object 1016 corresponding to a target time (e.g., a target time), an object 1017 corresponding to a period of sensing (e.g., a temperature change detection interval), an object 1018 corresponding to a current body temperature, and an object 1019 corresponding to start of measurement. The third target value means an amount of temperature change desired by a caregiver. The target time means an end time of monitoring of biometric information (e.g., the biometric information of FIG. 3A) of the patient.

In an embodiment, the electronic device may obtain body temperature setting information including at least one of the third target value, the target time, the period of sensing, and information about the current body temperature based on an input corresponding to each of the objects 1015, 1016, 1017, and 1018.

In an embodiment, when an input (e.g., a touch input) for the object 1019 corresponding to start of measurement is identified, the electronic device may store medication information and body temperature setting information input through the execution screen in the memory (e.g., the first memory 230 of FIG. 2 or the second memory 250 of FIG. 2). In an embodiment, when an input for the object 1019 corresponding to start of measurement is identified, the electronic device may transmit at least one of information about the third target value, the target time, and the period of sensing to an external device (e.g., the external device of FIG. 3A (e.g., a wearable device or a server)) through a communication module (e.g., the communication module 220 of FIG. 2).

According to an embodiment, the external device may transmit sensing data based on the period of sensing obtained from the electronic device. In an embodiment, when information about a period of sensing (e.g., 2 minutes) is input through the execution screen, the electronic device may transmit the input information about the period of sensing to an external device through a communication module (e.g., the communication module 220 of FIG. 2). A specific embodiment in which the electronic device identifies sensing data based on information about a period of sensing is described in detail with reference to FIG. 11 below.

FIG. 11 is a view illustrating a method of identifying sensing data based on a period of sensing according to an embodiment.

Referring to FIG. 11, an electronic device (e.g., the electronic device 200 of FIG. 2 (e.g., the processor 240)) according to an embodiment may receive sensing data (e.g., the sensing data of FIG. 3A) from an external device (e.g., the external device of FIG. 3A (e.g., a wearable device or a server)) through a communication module (e.g., the communication module 220 of FIG. 2) at a time corresponding to the period of sensing (e.g., the period of sensing of FIG. 10).

In an embodiment, as described with reference to FIG. 10, the electronic device may transmit information about a period of sensing input through the execution screen to an external device through the communication module. In an embodiment, the period of sensing may be a period where the electronic device receives sensing data, and the period of sensing may be different from a period where the external device measures data (or a measurement period). For example, the external device may measure data at 1-minute intervals, and the external device may transmit sensing data to the electronic device at 2-minute intervals based on the period of sensing received from the electronic device.

In an embodiment, sensing data transmitted at a time corresponding to the period of sensing may be transmitted as a data bundle. For example, a case where the external device obtains sensing data at 1-minute intervals and sensing data is transmitted to the electronic device at 2-minute intervals is assumed. The external device may obtain first sensing data (t1) 1110 corresponding to a first time 1101 and second sensing data (t2) 1120 corresponding to a second time 1102. The external device may transmit the first sensing data (t1) 1110 and the second sensing data (t2) 1120 to the electronic device at the second time 1102. Similarly, the external device may transmit third sensing data (t3) and fourth sensing data (t4) together to the electronic device, and may transmit fifth sensing data (t5) and sixth sensing data (t6) together to the electronic device. However, the disclosure is not limited thereto, and a period where the external device obtains sensing data and the period of sensing may have different values.

In an embodiment, sensing data transmitted at a time corresponding to the period of sensing may be an average value of sensing data corresponding to the period of sensing. For example, the external device may calculate an average value of the first sensing data (t1) 1110 and the second sensing data (t2) 1120. The external device may transmit the average value of the first sensing data (t1) 1110 and the second sensing data (t2) 1120 to the electronic device at the second time 1102.

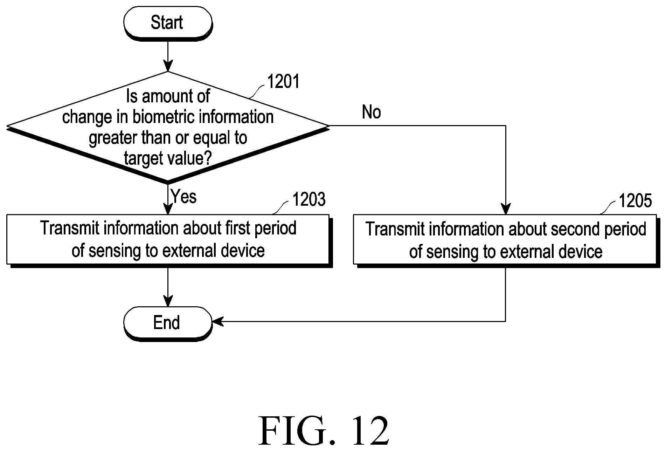

FIG. 12 is a flowchart illustrating a method of identifying sensing data based on a period of sensing according to an embodiment.

Referring to FIG. 12, an electronic device (e.g., the electronic device 200 of FIG. 2 (e.g., the processor 240)) according to an embodiment may transmit information about a changed period of sensing (e.g., the period of sensing of FIG. 10) to an external device (e.g., the external device of FIG. 3A (e.g., a wearable device or a server)) through a communication module (e.g., the communication module 220 of FIG. 2) based on an amount of change in the biometric information (e.g., the biometric information of FIG. 3A). The electronic device may set the period of sensing to a smaller value when the body temperature of the patient is high, which is a dangerous circumstance, so that sensing data (e.g., the sensing data of FIG. 3A) is obtained more frequently compared to a case where the body temperature of the patient is low.

The electronic device according to an embodiment may identify, in operation 1201, whether an amount of change in the biometric information is greater than or equal to a target value. In an embodiment, the target value may be any one of the first target value (e.g., the first target value of FIG. 4) or the second target value (e.g., the second target value of FIG. 4), but the disclosure is not limited thereto. In an embodiment, the electronic device may identify whether the amount of change in the biometric information is greater than or equal to the first target value as in operation 401 of FIG. 4. In an embodiment, the electronic device may also identify whether the amount of change in the biometric information is greater than or equal to the second target value as in operation 405 of FIG. 4.

In an embodiment, information about a target value (e.g., a reference temperature) according to age as illustrated in Table 1 may be stored in the memory (e.g., the first memory 230 of FIG. 2 or the second memory 250 of FIG. 2). In an embodiment, the electronic device may identify the age of the patient and may identify the reference temperature corresponding to the age based on Table 1. The electronic device may identify whether the amount of change in the biometric information is greater than or equal to the reference temperature.

**[Table 1]**

| Age | Reference temperature |
|---|---|
| Under 3 years old | 38 degrees |
| 3 years old or older | 37.8 degrees |

According to an embodiment, when the amount of change in the biometric information is greater than or equal to the target value (operation 1201 - YES), the electronic device may transmit, in operation 1203, information about a first period of sensing (e.g., 1 minute) to an external device through the communication module. According to an embodiment, when the amount of change in the biometric information is less than the target value (operation 1201 - NO), the electronic device may transmit, in operation 1205, information about a second period of sensing (e.g., 2 minutes) to an external device through the communication module. In an embodiment, a size of the first period of sensing may be less than a size of the second period of sensing.

According to the above-described example, the electronic device may obtain sensing data at a specific period of sensing. Further, the electronic device may obtain sensing data at the period of sensing set by the user in normal times, and may obtain sensing data at a short period of sensing in an urgent circumstance (e.g., when the amount of change in the biometric information is large), thereby enabling efficient battery use.

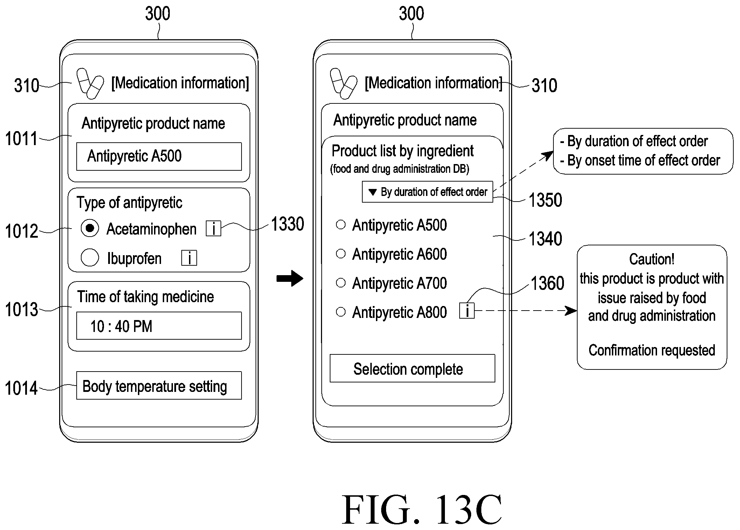

FIGS. 13A and 13B are views illustrating a method of identifying identification information of a patient according to an embodiment. FIG. 13C is a view illustrating a method of providing preset guide information according to an embodiment.

Referring to FIGS. 13A and 13B, an electronic device 300 (e.g., the electronic device 200 of FIG. 2 (e.g., the processor 240)) according to an embodiment may display an execution screen for identifying identification information of the patient (e.g., the identification information of the patient of FIG. 3A) on a touchscreen 310 (e.g., the touchscreen 210 of FIG. 2). In an embodiment, the identification information of the patient may include at least one of a name of the patient, a gender of the patient, a date of birth of the patient, and information about a weight of the patient.

In an embodiment, as illustrated in FIG. 13A, the electronic device 300 may display an execution screen for registering a patient through the touchscreen 310. In an embodiment, the execution screen for registering a patient may include at least one object for obtaining information about a name of the patient, a gender of the patient, a date of birth of the patient, and a weight of the patient. In an embodiment, when an input (e.g., a touch input) corresponding to an object 1310 for registering a patient is identified, the electronic device 300 may register (e.g., store in the first memory 230 of FIG. 2 or the second memory 250 of FIG. 2) the patient.

In an embodiment, as illustrated in FIG. 13B, the electronic device 300 may display an execution screen for selecting a patient through the touchscreen 310. In an embodiment, each of identification information of a plurality of patients may be stored in the memory (e.g., the first memory 230 of FIG. 2 or the second memory 250 of FIG. 2) of the electronic device. However, the disclosure is not limited thereto, and the identification information of the plurality of patients may also be stored in an external server (e.g., the server 108 of FIG. 1). In an embodiment, the electronic device 300 may display an execution screen for selecting any one of the plurality of patients through the touchscreen 310. In an embodiment, when an input (e.g., a touch input) corresponding to an object 1320 for selecting any one of the patients is identified, the electronic device 300 may display an execution screen for inputting (or modifying) the weight of the selected patient through the touchscreen 310.

FIG. 13C is a view illustrating a method of identifying medication information (e.g., the medication information of FIG. 2) according to an embodiment.

Referring to FIG. 13C, an electronic device 300 (e.g., the electronic device 200 of FIG. 2) according to an embodiment may display, as illustrated in FIG. 10, an execution screen including at least one of objects 1011, 1012, 1013, and 1014 through a touchscreen 310 (e.g., the touchscreen 210 of FIG. 2). In an embodiment, the object 1012 corresponding to a type of the medicine may include an object 1330 for selecting any one of medicines corresponding to a specific type.

In an embodiment, when a touch input for the object 1330 corresponding to a specific type (e.g., acetaminophen) is obtained, the electronic device 300 may display an object 1340 for selecting any one of a plurality of medicines (e.g., antipyretic A500, antipyretic A600, antipyretic A700, and antipyretic A800) corresponding to the specific type (e.g., acetaminophen) through the touchscreen 310.

In an embodiment, the object 1340 for selecting any one of the plurality of medicines may include objects 1350 and 1360. In an embodiment, the object 1340 may include an object 1350 for selecting a sort order of medicines. In an embodiment, the electronic device 300 may obtain an input (e.g., a touch input or a drag input) for selecting any one of a type sorted according to an order of duration of effect or a type sorted according to an order of a time when the medicine takes effect through the object 1350. In an embodiment, the electronic device 300 may obtain an input (e.g., a touch input) for identifying issue information about the medicine corresponding to the object 1360 through the object 1360.

In an embodiment, the electronic device 300 may obtain information about each of a plurality of types of medicines through an external device (e.g., the electronic device 102 of FIG. 1 or the server 108). In an embodiment, the information about the medicine may include different types of information including identification information of the medicine (e.g., a product name of the medicine), a type of the medicine (e.g., acetaminophen or ibuprofen), a recommended dosage, and issue information about the medicine. In an embodiment, the electronic device 300 may obtain information about the medicine from an external server corresponding to a food and drug administration through a communication module (e.g., the communication module 220 of FIG. 2).

In an embodiment, information about the medicine including information about a recommended dosage may also be stored in the memory (e.g., the memory 230 of FIG. 2 or the second memory 250 of FIG. 2). In an embodiment, the information about the recommended dosage may have stored therein information about a single dosage corresponding to an age and weight of the patient for each type of medicine. In an embodiment, information about a recommended dosage as illustrated in Table 2 below may be stored in the memory.

**[Table 2]**

| Age | Weight (kg) | Single dosage (ml) |
|---|---|---|
| 12 years old | 43 | 20 |
| 11 years old | 38-42.9 | 15 |
| 9-10 years old | 30-37.9 | 12.5 |
| 6-8 years old | 21-29.9 | 10 |
| 4-5 years old | 16-20.9 | 7.5 |
| 2-3 years old | 12-15.9 | 5 |
| 12-23 months | 10-11.9 | 3.5 |
| 4-11 months | 7-9.9 | 2.5 |

According to the above-described example, when medication is scheduled for a patient, a user may select a medicine considering information about a medicine scheduled to be taken. FIG. 14 is a flowchart illustrating a method of providing guide information corresponding to cross-dose according to an embodiment.

Referring to FIG. 14, an electronic device (e.g., the electronic device 200 of FIG. 2 (e.g., the processor 240)) according to an embodiment may identify, in operation 1401, whether the patient may take medicine based on medication information (e.g., the medication information of FIG. 2) and history information (e.g., the history information of FIG. 7). In an embodiment, the history information may include information about at least one medication event corresponding to the patient. In an embodiment, the information about the medication event may include at least one of information about a type of medication, a type of the medicine, a product name of the medicine, a time of taking medicine, a dosage, a time when the medicine takes effect, and a duration of effect of the medicine, but the disclosure is not limited thereto. In an embodiment, the history information may be stored in the memory (e.g., the memory 230 of FIG. 2 or the second memory 250 of FIG. 2), but the disclosure is not limited thereto. The history information is described in detail with reference to FIG. 16.

In an embodiment, whether the patient may take medicine may be identified as whether a reference dosage for a medicine of a specific type (e.g., acetaminophen) is exceeded within a designated time period (e.g., a time period 24 hours before the current time). In an embodiment, when medication information is input, the electronic device may identify whether the patient overdoses (or exceeds a reference dosage) a medicine of a specific type based on the history information. For example, a case where medication information for taking acetaminophen is input is assumed. The electronic device may identify the dosage of acetaminophen of the patient during a designated time period based on the history information.

When it is identified that the patient may not take medicine (operation 1401 - NO), the electronic device according to an embodiment may display, in operation 1403, guide information (e.g., the guide information corresponding to cross-dose of FIG. 6) corresponding to cross-dose through a touchscreen (e.g., the touchscreen 210 of FIG. 2). In an embodiment, the guide information corresponding to cross-dose may include at least one of information for notifying that the reference dosage for a medicine of a specific type has been exceeded, or information for suggesting taking a medicine of a different type. In an embodiment, the electronic device may also output a vibration-type alarm through a haptic module (e.g., the haptic module 179 of FIG. 1) or an audio-type alarm through a speaker (e.g., the sound output module 155 of FIG. 1) along with the guide information corresponding to cross-dose.

Based on identifying that the patient may take medicine (operation 1401 - YES), the electronic device according to an embodiment may update, in operation 1405, the history information to include the medication information.

According to the above-described example, when the medicine scheduled to be taken by the patient is likely to exceed the reference dosage, a user may receive a guide thereon and may receive a guide to take a medicine of a type different from the type of the medicine scheduled to be taken.

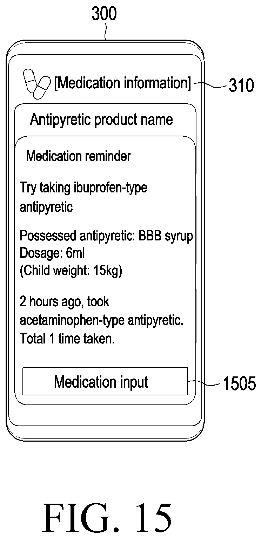

FIG. 15 is a view illustrating a method of providing guide information corresponding to cross-dose according to an embodiment.

Referring to FIG. 15, an electronic device 300 (e.g., the electronic device 200 of FIG. 2 (e.g., the processor 240)) according to an embodiment may display guide information (e.g., the guide information corresponding to cross-dose of FIG. 14) corresponding to cross-dose through a touchscreen 310 (e.g., the touchscreen 210 of FIG. 2).

In an embodiment, when it is identified that the patient may not take a medicine of a specific type (e.g., acetaminophen), the electronic device 300 may display information for suggesting taking a medicine of a different type (e.g., ibuprofen) through the touchscreen. In an embodiment, the information for suggesting taking a medicine of a different type may include a product name of a medicine of a specific type (e.g., ibuprofen) currently possessed by the caregiver (e.g., BBB syrup), a recommended dosage (e.g., 6 ml), a weight of the patient (e.g., 15 kg), and a medication history of the patient. In an embodiment, the electronic device may identify information for suggesting taking a medicine based on information about a product of the medicine (e.g., the information about a product of the medicine of FIG. 13C) and history information (e.g., the history information of FIG. 7).

In an embodiment, when an input (e.g., a touch input) for an object 1505 corresponding to medication input is identified, the electronic device 300 may update the history information based on the information for suggesting taking a medicine. For example, the electronic device may identify the suggested information as medication information (e.g., the medication information of FIG. 2). The electronic device may update the history information to include the suggested information.

FIG. 16 is a view illustrating a method of providing history information according to an embodiment.

Referring to FIG. 16, an electronic device 300 (e.g., the electronic device 200 of FIG. 2 (e.g., the processor 240)) according to an embodiment may provide history information (e.g., the history information of FIG. 7).

In an embodiment, the electronic device 300 may display objects corresponding to a plurality of pieces of history information including an object 1601 corresponding to first history information, an object 1602 corresponding to second history information, and an object 1603 corresponding to third history information through a touchscreen 310 (e.g., the touchscreen 210 of FIG. 2).

In an embodiment, when a plurality of medication events corresponding to the patient occur, history information corresponding to each of the plurality of medication events may be displayed through the touchscreen 310. The history information may include information about a medication event. In an embodiment, the information about the medication event (e.g., the information about the medication event of FIG. 8) may include at least one of information about a type of medication, a type of the medicine, a product name of the medicine, a time of taking medicine, a dosage, a time when the medicine takes effect, and a duration of effect of the medicine, but the disclosure is not limited thereto. In an embodiment, the history information may be stored in the memory (e.g., the memory 230 of FIG. 2 or the second memory 250 of FIG. 2).

The electronic device 300 according to an embodiment may provide a recommended dosage (e.g., the recommended dosage of Table 1) based on any one of a current weight or age of the patient. In an embodiment, when an input (e.g., a touch input) corresponding to any one of the objects 1601, 1602, and 1603 is identified, the electronic device 300 may display an object 1604 corresponding to any one for which the input is identified through the touchscreen 310. In an embodiment, the object 1604 may include at least a portion of the history information corresponding to the object 1601.

In an embodiment, the object 1604 may include information about a single recommended dosage identified based on the current weight and current age of the patient. For example, the electronic device 300 may identify the current weight and current age of the patient based on the history information. The electronic device 300 may identify information about a single recommended dosage based on information about a product of the medicine (e.g., the information about a product of the medicine of FIG. 13C), the identified current weight, and the current age. When an input (e.g., a touch input) for an identification object 1605 is identified, the electronic device 300 may display the objects 1601, 1602, and 1603 through the touchscreen 310 as shown on the left side of FIG. 16.

In an embodiment, the electronic device 300 may also obtain information about each product of the medicine through a QR code.

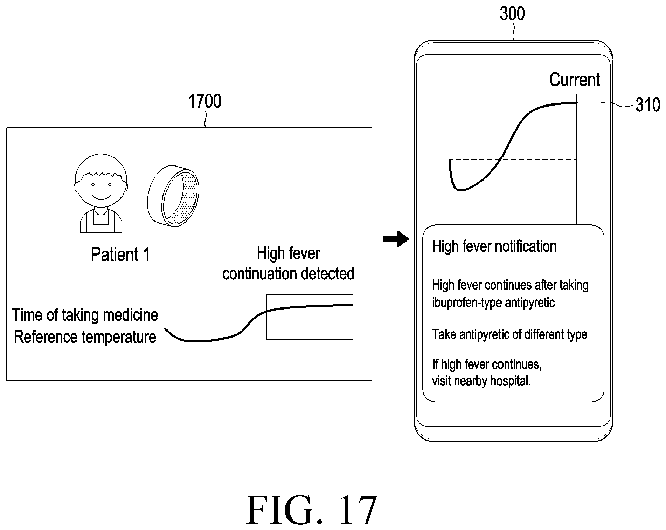

FIG. 17 is a view illustrating a method of providing preset guide information according to an embodiment.

Referring to FIG. 17, when a high fever of the patient is continuously detected 1700 even after medication, an electronic device 300 (e.g., the electronic device 200 of FIG. 2 (e.g., the processor 240)) according to an embodiment may provide guide information for notifying that the high fever of the patient continues.

In an embodiment, the electronic device 300 may identify that an amount of change in the biometric information (e.g., a body temperature) is greater than or equal to a target value (e.g., a first target value (e.g., the first target value of FIG. 4) or a second target value (e.g., the second target value of FIG. 4)) during a designated time period. In an embodiment, the designated time period may be a time period between a first time when a designated time (e.g., 2 hours) elapses from the time of taking medicine, and a second time when a designated time (e.g., 30 minutes) elapses from the first time, but the disclosure is not limited thereto.

In an embodiment, when it is identified that an amount of change in the biometric information (e.g., a body temperature) is greater than or equal to a target value during the designated time period, the electronic device 300 may display guide information for notifying that the high fever of the patient continues through a touchscreen 310 (e.g., the touchscreen 210 of FIG. 2). In an embodiment, the guide information for notifying that the high fever of the patient continues may include guide information for suggesting taking a medicine of a type (e.g., acetaminophen) different from the type (e.g., ibuprofen) of the medicine taken by the patient. In an embodiment, the electronic device 300 may also provide graph-type biometric information (e.g., a body temperature) along with the guide information for notifying that the high fever of the patient continues.

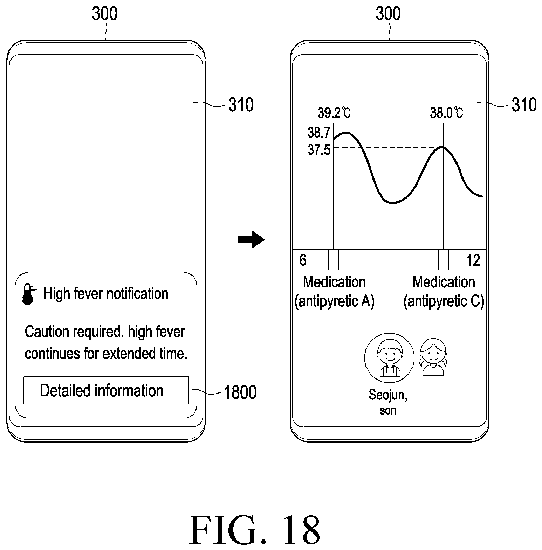

FIG. 18 is a view illustrating a method of providing preset guide information according to an embodiment.

Referring to FIG. 18, as illustrated in FIG. 17, when a high fever of the patient is continuously detected 1700 even after medication, an electronic device 300 (e.g., the electronic device 200 of FIG. 2 (e.g., the processor 240)) according to an embodiment may provide guide information for notifying that the high fever of the patient continues and an amount of change in the biometric information (e.g., body temperature change information).

In an embodiment, when it is identified that an amount of change in the biometric information (e.g., a body temperature) is greater than or equal to a target value during the designated time period, the electronic device 300 may display guide information for notifying that the high fever of the patient continues through a touchscreen 310 (e.g., the touchscreen 210 of FIG. 2). In an embodiment, the electronic device 300 may provide the guide information along with an alarm (e.g., the audio-type alarm of FIG. 5 or a vibration-type alarm). In an embodiment, the electronic device 300 may display an object 1800 for providing detailed information related to the guide information on the touchscreen 310.

In an embodiment, when an input (e.g., a touch input) corresponding to the object 1800 is identified, the electronic device 300 may display body temperature change information of the patient through the touchscreen 310. In an embodiment, the body temperature change information may include identification information of the patient with a continuous high fever and graph-type information corresponding to a change in the body temperature of the specific patient. In an embodiment, the information about the body temperature change may include a type of the medicine corresponding to a time of taking medicine of the specific patient and a body temperature corresponding to the time of taking medicine.

Objects of the disclosure are not limited to the foregoing, and other unmentioned objects would be apparent to one of ordinary skill in the art.

An electronic device 200; 300 according to an embodiment may include a touchscreen 210; 310, communication circuitry 220, first memory 230 storing instructions, at least one processor 240, and second memory 250.

According to an embodiment, the instructions may, when executed by the at least one processor 240, cause the electronic device 200; 300 to store information about a medicine administered to a patient and information of the administration time, obtained through the touchscreen 210; 310, as medication information in the second memory 250. According to an embodiment, the instructions may, when executed by the at least one processor 240, cause the electronic device 200; 300 to store first data related to the patient, received through the communication circuitry 220 from an external device, in the second memory 250.

According to an embodiment, the instructions may, when executed by the at least one processor 240, cause the electronic device 200; 300 to store second data related to the patient, received through the communication circuitry 220 from the external device, in the second memory 250.

According to an embodiment, the instructions may, when executed by the at least one processor 240, cause the electronic device 200; 300 to identify an amount of change in biometric information for the patient based on the first data and the second data.

According to an embodiment, the instructions may, when executed by the at least one processor 240, cause the electronic device 200; 300 to provide a notification based on the medication information stored in the second memory 250 and the identified amount of change in the biometric information.

According to an embodiment, each of the first data and the second data may include at least one of data of the temperature of the patient, data of the heart rate of the patient, or data of the oxygen saturation of the patient.

According to an embodiment, the electronic device 200; 300 may further include a camera.

According to an embodiment, the instructions may, when executed by the at least one processor 240, cause the electronic device 200; 300 to identify the medication information based on an input through the touchscreen 210; 310 or based on a QR code obtained through the camera.

According to an embodiment, the instructions may cause the identified medication information to be stored in the memory 230.

According to an embodiment, the instructions may, when executed by the at least one processor 240, cause the electronic device 200; 300, based on identifying that an amount of change in the biometric information is greater than or equal to a first target value, to display guide information for suggesting a visit to an emergency room through the touchscreen 210.

According to an embodiment, the instructions may cause guide information for suggesting continuous observation to be displayed through the touchscreen 210 based on identifying that the amount of change in the biometric information is greater than or equal to a second target value and less than the first target value.

According to an embodiment, the electronic device 200; 300 may further include a speaker.

According to an embodiment, the instructions may, when executed by the at least one processor 240, cause the electronic device 200; 300, based on identifying that an amount of change in the biometric information is greater than or equal to the first target value, to output a first alarm sound corresponding to a first period through the speaker.

According to an embodiment, the instructions may cause a second alarm sound corresponding to a second period to be output through the speaker based on identifying that the amount of change in the biometric information is greater than or equal to the second target value and less than the first target value, and the first period may be less than the second period.

According to an embodiment, the instructions may, when executed by the at least one processor 240, cause the electronic device 200; 300, based on identifying that an amount of change in the biometric information is less than the second target value, to identify whether a first time has passed from a time when the patient took medicine.

According to an embodiment, the instructions may cause guide information for indicating a need to take medicine to be displayed through the touchscreen 210 and an alarm sound to be output through the speaker based on identifying that the first time has passed from the time when the patient took medicine.

According to an embodiment, the instructions may, when executed by the at least one processor 240, cause the electronic device 200; 300, based on identifying that an amount of change in the biometric information is greater than or equal to the first target value, to transmit, through the communication circuitry 220, the biometric information and history information related to medication to a server corresponding to an emergency room.

According to an embodiment, the instructions may, when executed by the at least one processor 240, cause the electronic device 200; 300 to identify an estimated body temperature based on a body temperature input through the touchscreen 210 and the first data.

According to an embodiment, the instructions may cause guide information to be displayed through the touchscreen 210 based on identifying that the estimated body temperature is greater than or equal to a target temperature.

According to an embodiment, the instructions may, when executed by the at least one processor 240, cause the electronic device 200; 300 to transmit, through the communication circuitry 220, information about a period of sensing to the external device.

According to an embodiment, the instructions may cause the first data or the second data, received through the communication circuitry 220, to be identified at a time corresponding to the period of sensing.

According to an embodiment, the instructions may, when executed by the at least one processor 240, cause the electronic device 200; 300, based on identifying that an amount of change in the biometric information is greater than or equal to a target value, to transmit, through the communication circuitry 220, information about a first period of sensing to the external device.

According to an embodiment, the instructions may cause information about a second period of sensing to be transmitted to the external device through the communication circuitry 220 based on identifying that an amount of change in the biometric information is less than the target value, and a size of the first period of sensing may be less than a size of the second period of sensing.

According to an embodiment, the instructions may, when executed by the at least one processor 240, cause the electronic device 200; 300 to identify whether the patient may take medicine based on the medication information and history information. The medication information may further include identification information of the patient.

According to an embodiment, the instructions may cause guide information corresponding to cross-dose to be displayed through the touchscreen 210 based on identifying that the patient may not take medicine.

According to an embodiment, the instructions may cause the history information to be updated to include the medication information based on identifying that the patient may take medicine.

According to an embodiment, the instructions may, when executed by the at least one processor 240, cause the electronic device 200; 300 to display preset guide information through the touchscreen 210 based on identifying that an amount of change in the biometric information is greater than or equal to a target value during a first time period. The biometric information may include at least one of a temperature, a heart rate, and an oxygen saturation.

According to an embodiment, the instructions may, when executed by the at least one processor 240, cause the electronic device 200; 300 to display the heart rate and the oxygen saturation along with guide information through the touchscreen 210.

According to an embodiment, a method of operating an electronic device 200; 300 may include storing information about a medicine administered to a patient and information of the administration time, obtained through a touchscreen 210; 310, as medication information in second memory 250.

According to an embodiment, the method may include storing first data related to the patient, received from an external device, in the second memory 250.

According to an embodiment, the method may include storing second data related to the patient, received from the external device, in the second memory 250.

According to an embodiment, the method may include identifying an amount of change in biometric information for the patient based on the first data and the second data.

According to an embodiment, the method may include providing a notification based on the medication information stored in the second memory 250 and the identified amount of change in the biometric information.

According to an embodiment, each of the first data and the second data may include at least one of data of the temperature of the patient, data of the heart rate of the patient, or data of the oxygen saturation of the patient.

According to an embodiment, storing the input medication information in the memory 230 may include identifying the medication information based on a QR code obtained through a camera.

According to an embodiment, storing the input medication information in the memory 230 may include storing the identified medication information in the memory 230.

According to an embodiment, providing the notification related to medication may include, based on identifying that an amount of change in the biometric information is greater than or equal to a first target value, displaying guide information for suggesting a visit to an emergency room.

According to an embodiment, providing the notification may include, based on identifying that an amount of change in the biometric information is greater than or equal to a second target value and less than the first target value, displaying guide information for suggesting continuous observation, and the second target value may be different from the first target value.

According to an embodiment, providing the notification may include, based on identifying that an amount of change in the biometric information is greater than or equal to the first target value, outputting a sound of a first alarm corresponding to a first period.

According to an embodiment, the method may include, based on identifying that an amount of change in the biometric information is greater than or equal to the second target value and less than the first target value, outputting a sound of a second alarm corresponding to a second period, and a size of the first period may be less than a size of the second period.

According to an embodiment, providing the notification may include, based on identifying that an amount of change in the biometric information is less than the second target value, identifying whether a first time has passed from a time when the patient took medicine.

According to an embodiment, providing the notification related to medication may include, based on identifying that the first time has passed from the time when the patient took medicine, displaying guide information for indicating a need to take medicine through the touchscreen 210 and outputting a sound of an alarm.

According to an embodiment, the method may include, based on identifying that an amount of change in the biometric information is greater than or equal to the first target value, transmitting the biometric information and history information related to medication to a server corresponding to an emergency room.

According to an embodiment, identifying an amount of change in the biometric information may include identifying an estimated body temperature based on a body temperature input through a touchscreen 210 and the sensing data.

According to an embodiment, providing the notification related to medication may include, based on identifying that the estimated body temperature is greater than or equal to a target temperature, displaying guide information.

According to an embodiment, in a storage medium 230 storing computer-readable instructions, the instructions may, when the instructions are executed by at least one processor 240 of an electronic device 200; 300, cause the electronic device 200; 300 to store information about a medicine administered to a patient and information of the administration time, obtained through a touchscreen 210; 310, as medication information in second memory 250.

According to an embodiment, the instructions may, when executed by the at least one processor 240 of an electronic device 200; 300, cause the electronic device 200; 300 to store first data related to the patient, received through communication circuitry 220 from an external device, in the second memory 250.

According to an embodiment, the instructions may, when executed by the at least one processor 240 of an electronic device 200; 300, cause the electronic device 200; 300 to store second data related to the patient, received through the communication circuitry 220 from the external device, in the second memory 250.

According to an embodiment, the instructions may, when executed by the at least one processor 240 of an electronic device 200; 300, cause the electronic device 200; 300 to identify an amount of change in biometric information for the patient based on the first data and the second data.

According to an embodiment, the instructions may, when executed by the at least one processor 240 of an electronic device 200; 300, cause the electronic device 200; 300 to provide a notification based on the medication information stored in the second memory 250 and the identified amount of change in the biometric information.

Effects obtainable from the disclosure are not limited to the above-mentioned effects, and other effects not mentioned may be apparent to one of ordinary skill in the art.

As used herein, the term "if" may be interpreted to mean "when," "upon," "in response to determining," or "in response to detecting" according to the context. Similarly, "when determining that" or "when [a stated condition or event] is detected" may optionally be interpreted to mean "upon determining," "in response to determining," "upon detecting [the stated condition or event]," or "in response to detecting [the stated condition or event]."

The above-described devices may be implemented as hardware components, software components, and/or in a combination thereof. For example, the devices and components described herein may be implemented using one or more general-purpose or specific-purpose computers, such as processors, controllers, arithmetic logic units (ALUs), digital signal processors, micro-computers, field programmable gate arrays (FPGAs), programmable logic units (PLUs), micro-processors, any other devices capable of executing and responding to instructions. The processing device (or processing circuit) may perform an operating system (OS) and one or more software applications performed on the OS. The processing device or processor may access, store, manipulate or control, process, and generate data in response to the execution of the software. For illustration purposes, the processing device or processor may be a single one but it will be appreciated by one of ordinary skill in the art that a processing device may include a plurality of processing elements and/or a plurality of types of processing elements. For example, the processing device may include a plurality of processors or a single processor and a single controller. The server or device may have other various processing configurations, such as parallel processors.

The software may include computer programs, codes, instructions, or combinations of one or more thereof and may configure the processing device as it is operated as desired or may instruct the processing device independently or collectively. The software and/or data may be embodied in any type of machine, component, physical device, computer storage medium, or device so as to provide instructions or data to the processing device or to be interpreted by the processing device. The software may be distributed over computer systems connected together via a network to be distributively stored or executed. The software and data may be stored in one or more computer readable recording media.

The methods according to the embodiments may be implemented in the form of programming commands executable by various computer means, and the programming commands may be recorded in a computer-readable medium. In this case, the medium may continuously store computer-executable programs or temporarily store them for execution or download. Further, the medium may be a variety of recording or storage means in the form of a single piece of hardware or a combination of multiple pieces of hardware and, rather than being limited to a medium directly connected to a computer system, may be distributed over a network. Examples of the medium may include, but is not limited to, magnetic media, such as hard disks, floppy disks or magnetic tapes, optical recording media, such as CD-ROMs or DVDs, magneto-optical media, such as floptical disks, and ROMs, RAMs, or flash memories, or any other types of media configured to store program instructions. Further, examples of other media may include app stores that distribute applications, websites that supply or distribute various pieces of software, and recording media or storage media managed by servers.

Although the disclosure is shown and described in connection with embodiments, it will be easily appreciated by one of ordinary skill in the art that various changes or modifications may be made without departing from the scope of the disclosure. For example, although the techniques described herein are performed in a different order from those described herein and/or the components of the above-described structure or device are coupled, combined, or assembled in a different form from those described herein, or some components are replaced with other components or equivalents thereof, a proper result may be achieved.

Hence, other implementations, other embodiments, and equivalents to the claims also belong to the scope of the claims described below.

The electronic device according to various embodiments of the disclosure may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (200; 300) comprising:
a touchscreen (210; 310);
communication circuitry (220);
first memory (230) storing instructions;
second memory (250);
at least one processor (240); and
wherein the instructions, when executed by the at least one processor (240), cause the electronic device (200; 300) to:
store information about a medicine administered to a patient and information of the administration time, obtained through the touchscreen, as medication information in the second memory (250);
store first data related to the patient, received through the communication circuitry (220) from an external device, in the second memory (250);
store second data related to the patient, received through the communication circuitry (220) from the external device, in the second memory (250);
identify an amount of change in biometric information for the patient based on the first data and the second data; and
based on the medication information stored in the second memory (250) and the identified amount of change in the biometric information, provide a notification.

2. The electronic device (200; 300) of claim 1, wherein each of the first data and the second data comprises:
at least one of data of a temperature of the patient, data of a heart rate of the patient or data of an oxygen saturation of the patient.

3. The electronic device (200; 300) of claim 1 or 2, further comprising:
a camera (180),
wherein the instructions, when executed by the at least one processor (240), cause the electronic device (200; 300) to:
obtain the medication information based on an input through the touchscreen (210; 310) or based on a quick response (QR) code obtained through the camera (180).

4. The electronic device (200; 300) of any one of claims 1 to 3,
wherein the instructions, when executed by the at least one processor (240), cause the electronic device (200; 300) to:
based on identifying that the amount of change in the biometric information is greater than or equal to a first target value, display, through the touchscreen (210; 310), guide information for suggesting a visit to an emergency room, and
based on identifying that the amount of change in the biometric information is greater than or equal to a second target value and less than the first target value, display, through the touchscreen (210; 310), guide information for suggesting a continuous observation.

5. The electronic device (200; 300) of any one of claims 1 to 4, further comprising:
a speaker (155),
wherein the instructions, when executed by the at least one processor (240), cause the electronic device (200; 300) to:
based on identifying that the amount of change in the biometric information is greater than or equal to the first target value, output, through the speaker (155), a first alarm sound corresponding to a first period, and
based on identifying that the amount of change in the biometric information is greater than or equal to the second target value and less than the first target value, output, through the speaker (155), a second alarm sound corresponding to a second period, wherein the first period is less than the second period.

6. The electronic device (200; 300) of any one of claims 1 to 5,
wherein the instructions, when executed by the at least one processor (240), cause the electronic device (200; 300) to:
based on identifying that the amount of change in the biometric information is less than the second target value, identify whether a first time has passed from a time when the patient took medicine, and
based on identifying that the first time has passed from the time when the patient took medicine, display, through the touchscreen (210; 310), guide information for indicating a need to take medicine, and output, through the speaker (155), an alarm sound.

7. The electronic device (200; 300) of any one of claims 1 to 6,
wherein the instructions, when executed by the at least one processor (240), cause the electronic device (200; 300) to:
based on identifying that the amount of change in the biometric information is greater than or equal to the first target value, transmit, through the communication circuitry (220), the biometric information and history information related to medication to a server corresponding to an emergency room.

8. The electronic device (200; 300) of any one of claims 1 to 7,
wherein the instructions, when executed by the at least one processor (240), cause the electronic device (200; 300) to:
based on a body temperature input through the touchscreen (210; 310) and the first data, identify an estimated body temperature, and
based on identifying that the estimated body temperature is greater than or equal to a target temperature, display, through the touchscreen (210; 310), guide information.

9. The electronic device (200; 300) of any one of claims 1 to 8,
wherein the instructions, when executed by the at least one processor (240), cause the electronic device (200; 300) to:
transmit, through the communication circuitry (220), information about a period of sensing to the external device, and
identify the first data or the second data, received through the communication circuitry (220), at a time corresponding to the period of sensing.

10. The electronic device (200; 300) of any one of claims 1 to 9,
wherein the instructions, when executed by the at least one processor (240), cause the electronic device (200; 300) to:
based on identifying that the amount of change in the biometric information is greater than or equal to a target value, transmit, through the communication circuitry (220), information about a first period of sensing to the external device, and
based on identifying that the amount of change in the biometric information is less than the target value, transmit, through the communication circuitry (220), information about a second period of sensing to the external device,
wherein a size of the first period of sensing is less than a size of the second period of sensing.

11. The electronic device (200; 300) of any one of claims 1 to 10,
wherein the instructions, when executed by the at least one processor (240), cause the electronic device (200; 300) to:
based on the medication information and history information, identify whether the patient can take medicine, wherein the medication information further comprises identification information of the patient,
based on identifying that the patient cannot take medicine, display, through the touchscreen (210; 310), guide information corresponding to cross-dose, and
based on identifying that the patient can take medicine, update the history information to include the medication information.

12. The electronic device (200; 300) of any one of claims 1 to 11,
wherein the instructions, when executed by the at least one processor (240), cause the electronic device (200; 300) to:
based on identifying that the amount of change in the biometric information is greater than or equal to a target value during a first time period, display, through the touchscreen (210; 310), preset guide information.

13. The electronic device (200; 300) of any one of claims 1 to 12,
wherein the biometric information comprises at least one of a temperature, a heart rate or an oxygen saturation, and
wherein the instructions, when executed by the at least one processor (240), cause the electronic device (200; 300) to:
display, through the touchscreen (210; 310), the heart rate and the oxygen saturation along with guide information.

14. A method of operating an electronic device (200; 300), the method comprising:
storing information about a medicine administered to a patient and information of the administration time, obtained through a touchscreen (210; 310), as medication information in second memory (250);
storing first data related to the patient, received from an external device, in the second memory (250);
storing second data related to the patient, received from the external device, in the second memory (250);
identifying an amount of change in biometric information for the patient based on the first data and the second data; and
based on the medication information stored in the second memory (250) and the identified amount of change in the biometric information, providing a notification.

15. A storage medium (230) storing computer-readable instructions, wherein the instructions, when executed by at least one processor (240) of an electronic device (200; 300), cause the electronic device (200; 300) to:
store information about a medicine administered to a patient and information of the administration time, obtained through a touchscreen (210; 310), as medication information in second memory (250);
store first data related to the patient, received through communication circuitry (220) from an external device, in the second memory (250);
store second data related to the patient, received through the communication circuitry (220) from the external device, in the second memory (250);
identify an amount of change in biometric information for the patient based on the first data and the second data; and
based on the medication information stored in the second memory (250) and the identified amount of change in the biometric information, provide a notification.
